# EUROPEAN PATENT APPLICATION

(11) **EP 4 299 570 A1**
(43) Date of publication of application: **03.01.2024**
(21) Application number: 22758888.6
(22) Date of filing: 23.02.2022
(51) Int. Cl.: C07D 333/78, C07D 409/12, A61P 35/00

(54) **TRICYCLIC COMPOUND AS HIF2A INHIBITOR, PREPARATION METHOD THEREFOR AND APPLICATION THEREOF**

(30) Priority: 23.02.2021 CN 202110203800; 26.01.2022 CN 202210095335; 14.02.2022 CN 202210135655
(71) Applicant: Shanghai Jemincare Pharmaceuticals Co., Ltd., Pudong New Area, Shanghai 201203 (CN); Jiangxi Jemincare Group Co., Ltd., Nanchang, Jiangxi 330000 (CN)
(72) Inventor: BIE, Pingyan, Shanghai 201203 (CN); MOU, Jianfeng, Shanghai 201203 (CN); ZHANG, Jianmin, Shanghai 201203 (CN); WAN, Zhengyong, Shanghai 201203 (CN); YU, Qingfang, Shanghai 201203 (CN); CHEN, Zhe, Shanghai 201203 (CN); LI, Hongye, Shanghai 201203 (CN); PENG, Jianbiao, Shanghai 201203 (CN)
(74) Representative: Henderson, Helen Lee
(86) International application number: PCT/CN2022/077454
(87) International publication number: WO 2022/179524

(57) **Abstract**

A tricyclic compound as represented by formula (I), a preparation method therefor, and an application thereof as an HIF2a inhibitor.

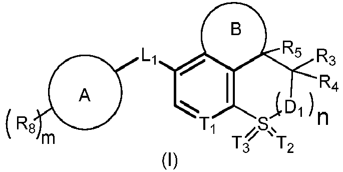

## Description

The present invention claims the right of the following priorities:
CN202110203800.3, application date: February 23, 2021;
CN202210095335.0, application date: January 26, 2022;
CN202210135655.4, application date: February 14, 2022.

### TECHNICAL FIELD

The present disclosure relates to a compound of formula (I), an optical isomer thereof, and a pharmaceutically acceptable salt thereof, and the use of the compound as an inhibitor of HIF2α.

### BACKGROUND

Renal cancer accounts for about 2% to 3% of adult malignant tumors, and 80% to 90% of adult renal malignant tumors. According to statistics, in 2018, there were 403,000 newly diagnosed patients with renal cancer worldwide, and 175,000 people died of it. The current incidence of renal cancer in China is about 4.0/100,000, while the incidence in cities is about 6.0/100,000. Based on this calculation, there are about 52,000 to 78,000 new patients with renal cancer every year in China, and the total number of patients with renal cancer is estimated to be 460,000 or more. Glioma accounts for 40 to 50% of brain tumors, which is the most common intracranial malignant tumor. Malignant gliomas are derived from glia, which are histologically heterogeneous and aggressive, and have a poor prognosis. Because renal cell carcinoma is not sensitive to radiotherapy and chemotherapy, targeted therapy has become the main treatment for advanced renal cancer in recent years, which significantly prolongs the survival of patients with renal cancer, especially those with metastatic advanced renal cancer. However, almost all patients who receive targeted therapy will develop drug resistance and tumor recurrence, and significant side effects are also present. Therefore, it is necessary to develop drugs targeting different tumorigenic genes for different signaling pathways and different drug resistance mechanisms for clinical selection in the order and combination of administration. Gradually achieve precise medicine for different patients, different disease subtypes, and stages of disease progression, to control the disease to the greatest extent, reduce side effects, and improve the quality of life of patients.

The VHL/HIF2α pathway dominates most renal carcinogenesis. VHL is the subunit of E3 ligase for binding targeted protein, responsible for protein degradation. VHL gene is a typical cancer suppressor gene, and dysfunction of the VHL gene can cause central nervous system hemangioma, renal cancer/renal cyst, retinal hemangioma, pheochromocytoma, pancreatic tumor, etc. Abnormalities in the VHL/HIF2α signaling pathway account for 90% or more in the case of renal cell carcinomas, especially in the case of clear cell carcinomas. VHL gene mutation, chromosomal deletion, and gene-level methylation modification can lead to the inactivation or activity reduction of VHL gene, and HIF2α cannot be degraded in time, accumulates and enters the nucleus to form a complex with HIF1β, resulting in the transcription of a series of downstream genes, such as vascular endothelial growth factor (VEGF), platelet-derived growth factor (PDGF), cyclin D, glucose transporter 1 (GLUT1), oxygen transport and metabolism, cell proliferation and migration, ultimately leading to the occurrence and metastasis of tumors. Therefore, the development of drugs targeting the VHL/HIF2α pathway can provide new and effective treatments for patients with renal cancer, among which Peloton's HIF2a inhibitor PT2977 has entered phase III clinical trials for the treatment of renal cancer. According to its mechanism of action, HIF2α inhibitors are also highly anticipated in the treatment of the rare disease, VHL syndrome.

Brain glioma is a tumor derived from the glial cells of the brain, accounting for 40 to 50% of brain tumors, and is the most common primary intracranial tumor. The annual incidence of brain glioma in China is 5/100,000 to 8/100,000, and the 5-year mortality rate is second only to pancreatic cancer and lung cancer among systemic tumors, among which glioblastoma (GBM) is the most common and deadly primary malignant brain tumor in adults. At present, the main treatment method is surgery, supplemented by radiotherapy and chemotherapy after operation, but the overall treatment effect is not ideal. The median survival time of newly diagnosed patients after receiving standard care is only 15 months, and their recurrence rate is high, and the median survival time after recurrence is only 5 to 7 months. It is clinically found that patients with high expression of HIF2α in glioblastoma have a worse prognosis. *In vitro* cytology experiments found that the expression of HIF2α was closely related to the tumorigenicity of glioma cells. PT2977 for the treatment of glioblastoma is currently in the clinical phase II, which proves that HIF2α inhibitors have certain effects in patients with this type of tumor, and can provide a new treatment strategy for this part of patients with extremely limited treatment options.

HIF2α inhibitors can also be used in the treatment of other tumors. Inhibition of HIF-2α protein can reduce the transcription and expression of factors related to angiogenesis, including vascular endothelial growth factor (VEGF), platelet-derived growth factor (PDGF), epidermal growth factor (EGF), etc., thereby inhibiting tumor angiogenesis. HIF2α inhibitors have the mechanism of action of anti-angiogenic drugs, so their use alone or in combination with immune checkpoint inhibitor drugs can also be extended to tumors with multiple indications for existing anti-angiogenic drugs, except for renal cancer, including lung cancer, colorectal cancer, ovarian cancer, breast cancer, cervical cancer, gastric cancer, liver cancer, thyroid cancer, and multiple myeloma, etc. In addition, studies have shown that HIF2α inhibitors act on the immune cell population in the tumor microenvironment, which can inhibit tumor growth by increasing the killing effect of T cells on tumors or reducing the effect of cells with immunosuppressive functions. It is suggested that HIF2α inhibitors alone or in combination with other drugs may have a therapeutic effect on liver cancer, pancreatic ductal carcinoma, lung squamous cell carcinoma, colon cancer, etc. In addition, the use of HIF2α inhibitors in the treatment of hemangiomas is also worthy of attention.

Finally, HIF2α also plays an important role in the occurrence and development of non-tumor fields such as pulmonary hypertension, reflux esophagitis, and inflammatory bowel disease. The successful development of HIF2a inhibitors will also provide new treatment options for these patients.

### CONTENT OF THE PRESENT INVENTION

The present disclosure provides a new class of tricyclic compounds. The compounds of the present disclosure show a good inhibitory effect in the luciferase assay and VEGF ELISA assay, and can be used in the treatment of various HIF2α-related diseases such as renal cancer and malignant glioma.

The present disclosure provides a compound of formula (I), an optical isomer thereof, or a pharmaceutically acceptable salt thereof, 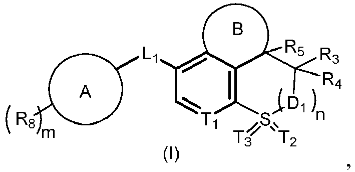
wherein ring A is selected from C₄₋₆ cycloalkyl, 4- to 6-membered heterocycloalkyl, phenyl, and 5- to 6-membered heteroaryl;
ring B is selected from C₅₋₆ cycloalkyl, 5- to 6-membered heterocyclyl, and 5- to 6-membered cycloalkenyl, and the C₅₋₆ cycloalkyl, 5- to 6-membered heterocyclyl, or 5- to 6-membered cycloalkenyl is optionally substituted by 1, 2, 3, or 4 R;
L₁ is selected from a single bond, -O-, -S-, and -N(R_{L})-;
T₁ is selected from -C(R_{T})- and -N-;
T₂ is selected from O, =NR₉, or T₂ is absent;
T₃ is selected from =NR₁₀ and O;
D₁ is independently selected from -C(R_{D1})₂- and -N(R_{D1})-;
R₃, R₄ are each independently selected from H, F, Cl, Br, and I;
R₅ is selected from H, OH, F, and NH₂;
R₈ is independently selected from H, F, Cl, Br, I, CN, C₁₋₆ alkyl, and C₁₋₆ alkoxy, and the C₁₋₆ alkyl or C₁₋₆ alkoxy is optionally substituted by 1, 2, or 3 Rsa;
R₉ is selected from H, CN, OH, C₁₋₆ alkyl, and C₃₋₆ cycloalkyl, and the C₁₋₆ alkyl and C₃₋₆ cycloalkyl are optionally substituted by 1, 2, or 3 R;
R₁₀ is selected from H, CN, OH, C₁₋₆ alkyl, and C₃₋₆ cycloalkyl, and the C₁₋₆ alkyl and C₃₋₆ cycloalkyl are optionally substituted by 1, 2, or 3 R;
R, R_{T}, R_{D1}, R_{L}, R₈ₐ are each independently selected from H, halogen, OH, NH₂, CN, 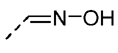 , C₁₋₆ alkyl, C₁₋₆ alkoxy, and C₂₋₆ alkenyl, and the C₁₋₆ alkyl, C₁₋₆ alkoxy, or C₂₋₆ alkenyl is optionally substituted by 1, 2, or 3 R';
R' is independently selected from H, halogen, OH, NH₂, CN, and C₁₋₆ alkyl;
m is independently 0, 1, 2, 3, or 4;
n is independently 0, 1, 2, or 3;
the 4- to 6-membered heterocycloalkyl, 5- to 6-membered heterocyclyl, or 5- to 6-membered heteroaryl contains 1, 2, or 3 heteroatoms or heteroatom groups independently selected from -O-, -NH-, -S-, -C(=O)-, -C(=O)O-, -S(=O)-, -S(=O)₂-, and N.

The present disclosure also provides a compound of formula (II), an optical isomer thereof, or a pharmaceutically acceptable salt thereof, 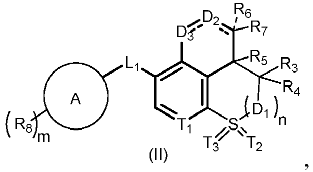
wherein ring A is selected from C₄₋₆ cycloalkyl, 4- to 6-membered heterocycloalkyl, phenyl, and 5- to 6-membered heteroaryl;
L₁ is selected from a single bond, -O-, -S-, and -N(R_{L})-;
T₁ is selected from -C(R_{T})- and -N-;
T₂ is selected from O, =NR₉, or T₂ is absent;
T₃ is selected from =NR₁₀ and O;
D₁ is independently selected from -C(R_{D1})₂- and -N(R_{D1})-;
when 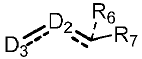 is 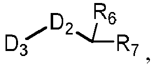 D₂ and D₃ are each independently selected from a single bond, -O-, -N(R)-, -C(R)₂-, -C(=R)-, -C(=O)-, and -C(=NR)-, and R₆, R₇ are each independently selected from H, F, Cl, Br, and I;
when 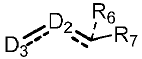 is 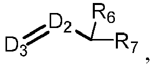 D₂ and D₃ are each independently selected from -C(R)- and N, and R₆, R₇ are each independently selected from H, F, Cl, Br, and I;
when 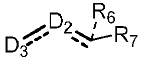 is 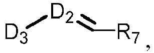 D₂ is independently selected from -C(R)- and N, D₃ is independently selected from a single bond, -O-, -N(R)-, -C(R)₂-, -C(=R)-, -C(=O)-, and -C(=NR)-, and R₇ is independently selected from H, F, Cl, Br, and I;
R₃, R₄ are each independently selected from H, F, Cl, Br, and I;
R₅ is selected from H, OH, F, and NH₂;
R₈ is independently selected from H, F, Cl, Br, I, CN, C₁₋₆ alkyl, and C₁₋₆ alkoxy, and the C₁₋₆ alkyl or C₁₋₆ alkoxy is optionally substituted by 1, 2, or 3 Rsa;
R₉ is selected from H, CN, OH, C₁₋₆ alkyl, and C₃₋₆ cycloalkyl, and the C₁₋₆ alkyl and C₃₋₆ cycloalkyl are optionally substituted by 1, 2, or 3 R;
R₁₀ is selected from H, CN, OH, C₁₋₆ alkyl, and C₃₋₆ cycloalkyl, and the C₁₋₆ alkyl and C₃₋₆ cycloalkyl are optionally substituted by 1, 2, or 3 R;
R, R_{T}, R_{D1}, R_{L}, R₈ₐ are each independently selected from H, halogen, OH, NH₂, CN, 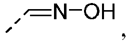 C₁₋₆ alkyl, C₁₋₆ alkoxy, and C₂₋₆ alkenyl, and the C₁₋₆ alkyl, C₁₋₆ alkoxy, or C₂₋₆ alkenyl is optionally substituted by 1, 2, or 3 R';
R' is independently selected from H, halogen, OH, NH₂, CN, and C₁₋₆ alkyl;
m is independently 0, 1, 2, 3, or 4;
n is independently 0, 1, 2, or 3;
the 4- to 6-membered heterocycloalkyl or 5- to 6-membered heteroaryl contains 1, 2, or 3 heteroatoms or heteroatom groups independently selected from -O-, -NH-, -S-, -C(=O)-, - C(=O)O-, -S(=O)-, -S(=O)₂-, and N.

The present disclosure also provides a compound of formula (II-A) or formula (II-B), an optical isomer thereof, or a pharmaceutically acceptable salt thereof, 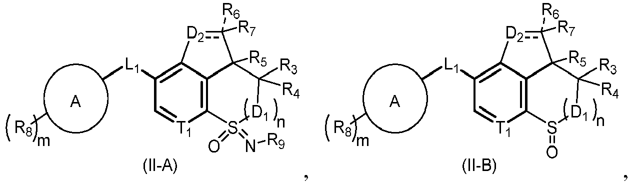
wherein ring A is selected from C₄₋₆ cycloalkyl, 4- to 6-membered heterocycloalkyl, phenyl, and 5- to 6-membered heteroaryl;
L₁ is selected from a single bond, -O-, -S-, and -N(R_{L})-;
T₁ is selected from -C(R_{T})- and -N-;
D₁ is independently selected from -C(R_{D1})₂- and -N(R_{D1})-;
when 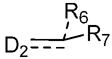 is 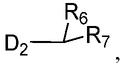 D₂ is selected from -O-, -N(R)-, -C(R)₂-, -C(=R)-, -C(=O)-, and -C(=NR)-, and R₆, R₇ are each independently selected from H, F, Cl, Br, and I;
when 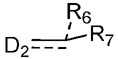 is 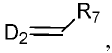 D₂ is selected from -C(R)- and N, and R₇ is selected from H, F, Cl, Br, and I;
R₃, R₄ are each independently selected from H, F, Cl, Br, and I;
R₅ is selected from H, OH, F, and NH₂;
Rs is independently selected from H, F, Cl, Br, I, CN, C₁₋₆ alkyl, and C₁₋₆ alkoxy, and the C₁₋₆ alkyl or C₁₋₆ alkoxy is optionally substituted by 1, 2, or 3 Rsa;
R₉ is selected from H, CN, OH, C₁₋₆ alkyl, and C₃₋₆ cycloalkyl, and the C₁₋₆ alkyl and C₃₋₆ cycloalkyl are optionally substituted by 1, 2, or 3 R;
R, R_{T}, R_{D1}, R_{L}, R₈ₐ are each independently selected from H, halogen, OH, NH₂, CN, 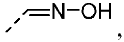 C₁₋₆ alkyl, C₁₋₆ alkoxy, and C₂₋₆ alkenyl, and the C₁₋₆ alkyl, C₁₋₆ alkoxy, or C₂₋₆ alkenyl is optionally substituted by 1, 2, or 3 R';
R' is independently selected from H, halogen, OH, NH₂, CN, and C₁₋₆ alkyl;
m is independently 0, 1, 2, 3, or 4;
n is independently 0, 1, 2, or 3;
the 4- to 6-membered heterocycloalkyl or 5- to 6-membered heteroaryl contains 1, 2, or 3 heteroatoms or heteroatom groups independently selected from -O-, -NH-, -S-, -C(=O)-, - C(=O)O-, -S(=O)-, -S(=O)₂-, and N.

In some embodiments of the present disclosure, for the compound of formula (I), the optical isomer thereof, or the pharmaceutically acceptable salt thereof, wherein the compound is represented by formula (III-A), 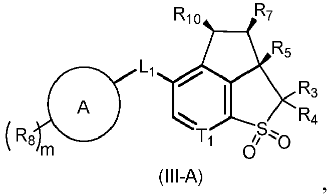
wherein ring A is selected from C₄₋₆ cycloalkyl, 4- to 6-membered heterocycloalkyl, phenyl, and 5- to 6-membered heteroaryl;
L₁ is selected from a single bond, -O-, -S-, and -N(R_{L})-;
T₁ is selected from -C(R_{T})- and -N-;
R₇, R₁₀ are each independently selected from H, F, Cl, Br, and I;
R₃, R₄ are each independently selected from H, F, Cl, Br, and I;
R₅ is selected from H, OH, F, and NH₂;
R₈ is independently selected from H, F, Cl, Br, I, CN, C₁₋₆ alkyl, and C₁₋₆ alkoxy, and the C₁₋₆ alkyl or C₁₋₆ alkoxy is optionally substituted by 1, 2, or 3 Rsa;
R_{T}, R_{L}, R₈ₐ are each independently selected from H, halogen, OH, NH₂, CN, 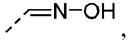 C₁₋₆ alkyl, C₁₋₆ alkoxy, and C₂₋₆ alkenyl, and the C₁₋₆ alkyl, C₁₋₆ alkoxy, or C₂₋₆ alkenyl is optionally substituted by 1, 2, or 3 R';
R' is independently selected from H, halogen, OH, NH₂, CN, and C₁₋₆ alkyl;
m is independently 0, 1, 2, 3, or 4;
the 4- to 6-membered heterocycloalkyl or 5- to 6-membered heteroaryl contains 1, 2, or 3 heteroatoms or heteroatom groups independently selected from -O-, -NH-, -S-, -C(=O)-, - C(=O)O-, -S(=O)-, -S(=O)₂-, and N.

In some embodiments of the present disclosure, the R₈ is selected from H, F, Cl, Br, I, and CN, and other variables are as defined in the present disclosure.

In some embodiments of the present disclosure, the R₉ is selected from H, CN, OH, Me, Et, 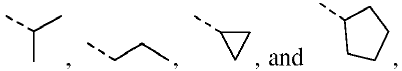 and the Me, Et, 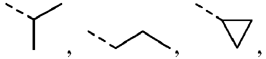 or 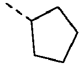 is optionally substituted by 1, 2, or 3 R, and other variables are as defined in the present disclosure.

In some embodiments of the present disclosure, the R₉ is selected from H, CN, OH, Me, Et, 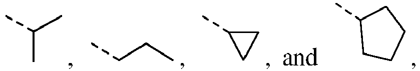 and other variables are as defined in the present disclosure.

In some embodiments of the present disclosure, the R₁₀ is selected from H, CN, OH, Me, Et, 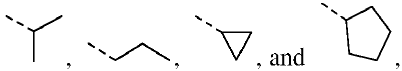 and the Me, Et, 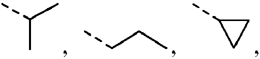 or 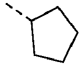 is optionally substituted by 1, 2, or 3 R, and other variables are as defined in the present disclosure.

In some embodiments of the present disclosure, the R₁₀ is selected from H, CN, OH, Me, Et, 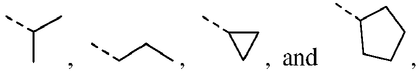 and other variables are as defined in the present disclosure.

In some embodiments of the present disclosure, the ring A is selected from phenyl, pyridyl, pyridazinyl, cyclobutyl, cyclopentyl, and cyclohexyl, and other variables are as defined in the present disclosure.

In some embodiments of the present disclosure, the structural moiety 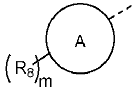 is selected from 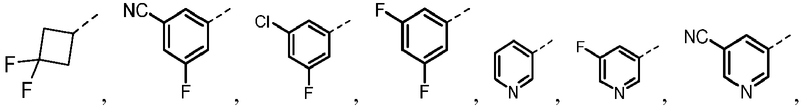 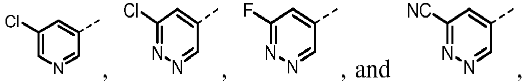 and other variables are as defined in the present disclosure.

In some embodiments of the present disclosure, the R, R_{T}, R_{D1}, R_{L}, R₈ₐ are independently selected from H, F, Cl, Br, I, CN, OH, 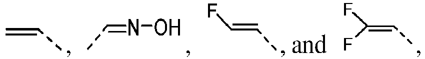 and other variables are as defined in the present disclosure.

In some embodiments of the present disclosure, the ring B is selected from cyclopentyl, cyclopentenyl, cyclohexyl, cyclohexenyl, oxocyclohexyl, tetrahydro-2H-pyran-2-keto, piperidin-2-keto, tetrahydro-2H-pyranyl, and piperidinyl, and the cyclopentyl, cyclopentenyl, cyclohexyl, cyclohexenyl, oxocyclohexyl, tetrahydro-2H-pyran-2-keto, piperidin-2-keto, tetrahydro-2H-pyranyl, or piperidinyl is optionally substituted by 1, 2, 3, or 4 R, and other variables are as defined in the present disclosure.

In some embodiments of the present disclosure, the ring B is selected from 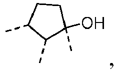 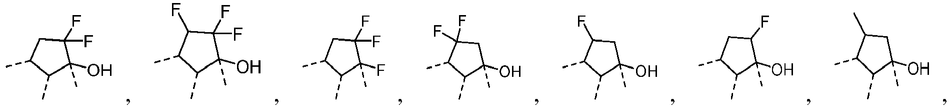 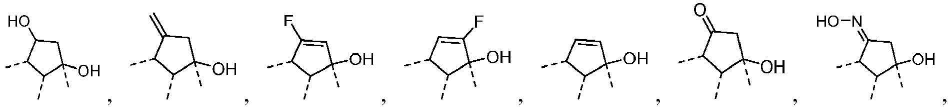 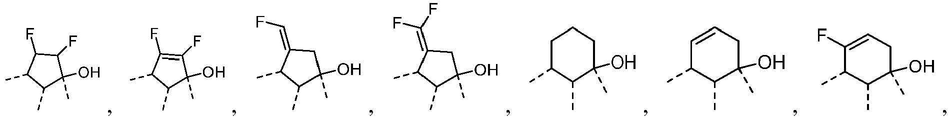 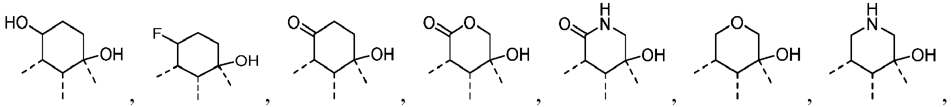 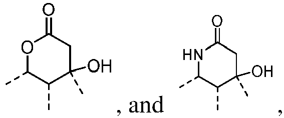 and other variables are as defined in the present disclosure.

In some embodiments of the present disclosure, the ring B is selected from 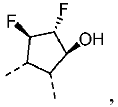 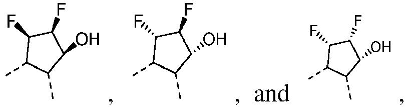 and other variables are as defined in the present disclosure.

In some embodiments of the present disclosure, the structural moiety 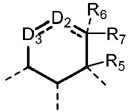 is selected from 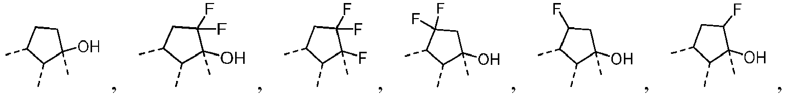 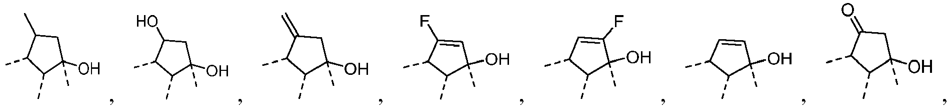 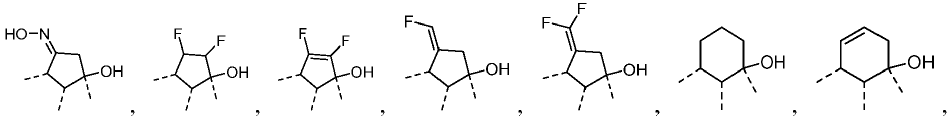 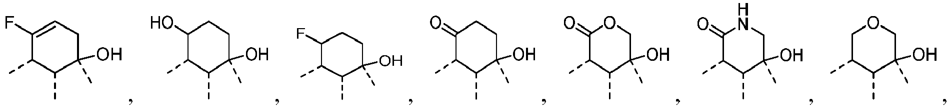 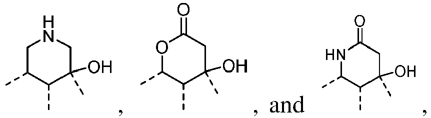 and other variables are as defined in the present disclosure.

In some embodiments of the present disclosure, the structural moiety 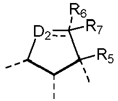 is selected from 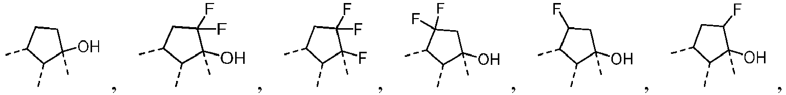 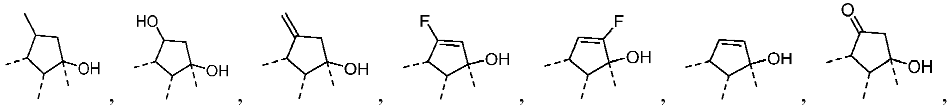 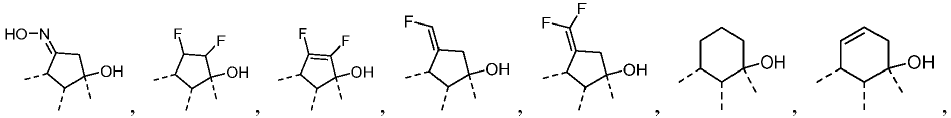 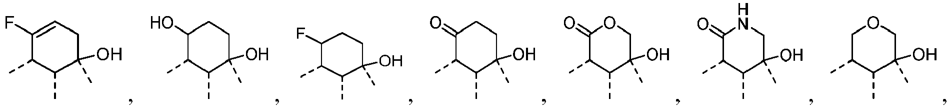 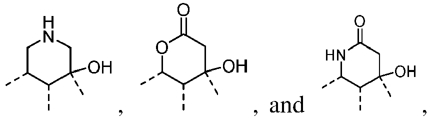 and other variables are as defined in the present disclosure.

In some embodiments of the present disclosure, the structural moiety 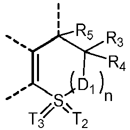 is selected from 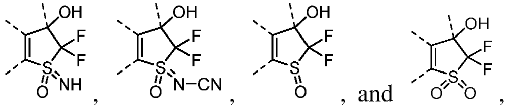 and other variables are as defined in the present disclosure.

In some embodiments of the present disclosure, the structural moiety 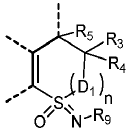 is selected from 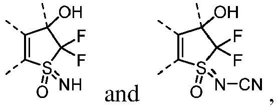 and other variables are as defined in the present disclosure.

In some embodiments of the present disclosure, the structural moiety 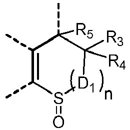 is selected from 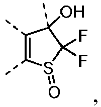 and other variables are as defined in the present disclosure.

In some embodiments of the present disclosure, the structural moiety 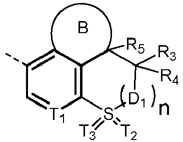 is selected from 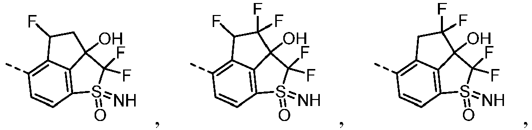 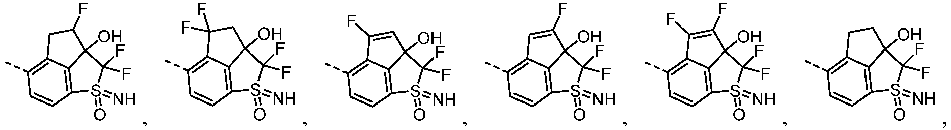 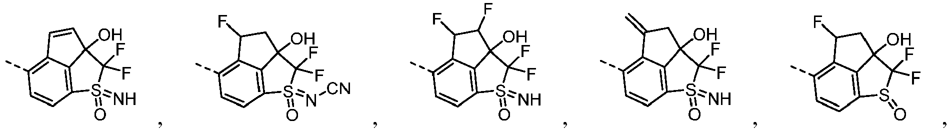 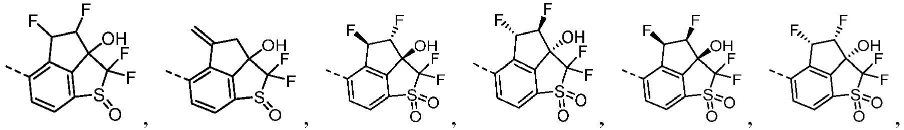 and other variables are as defined in the present disclosure.

In some embodiments of the present disclosure, the structural moiety 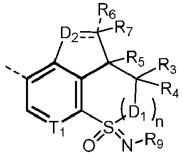 is selected from 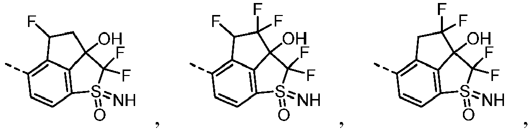 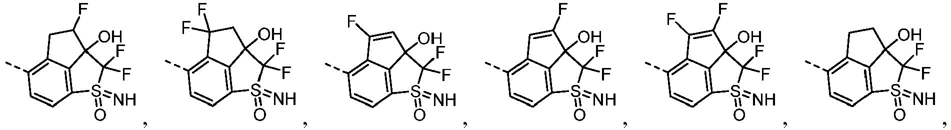 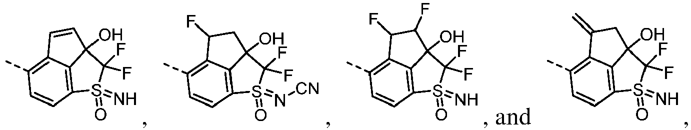 and other variables are as defined in the present disclosure.

In some embodiments of the present disclosure, the structural moiety 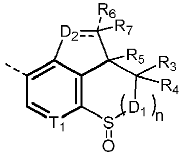 is selected from 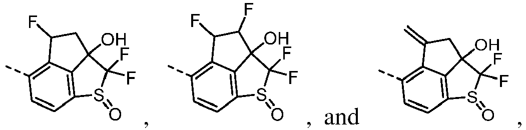 and other variables are as defined in the present disclosure.

The present disclosure also provides a compound of the following formula, an optical isomer thereof, or a pharmaceutically acceptable salt thereof, selected from:

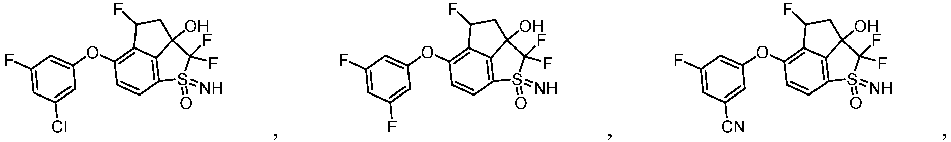

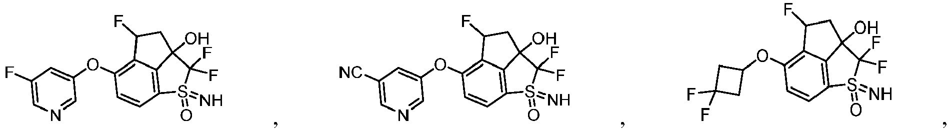

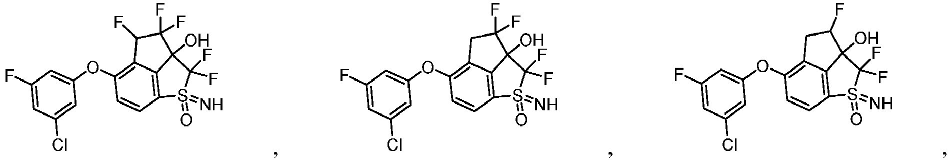

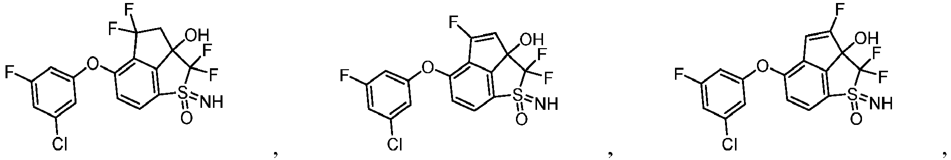

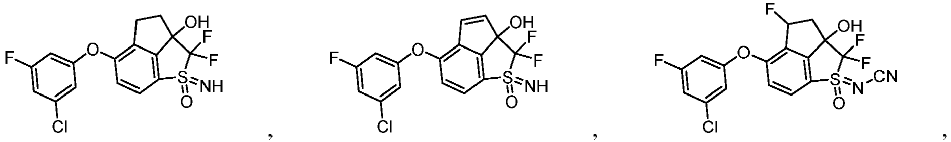

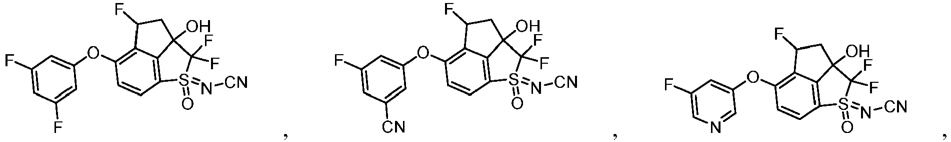

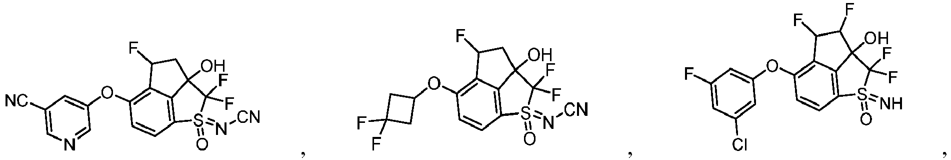

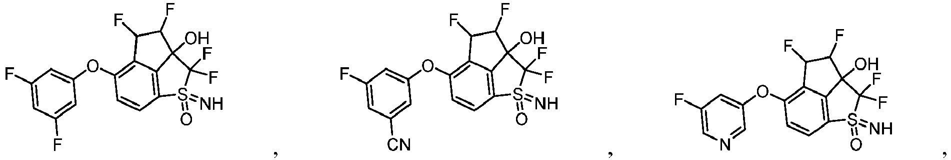

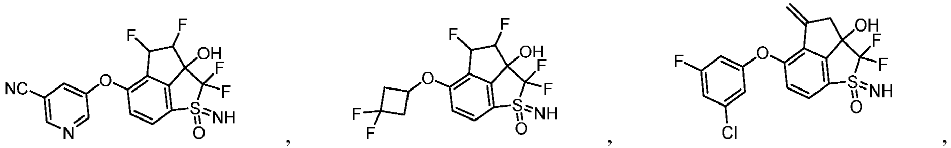

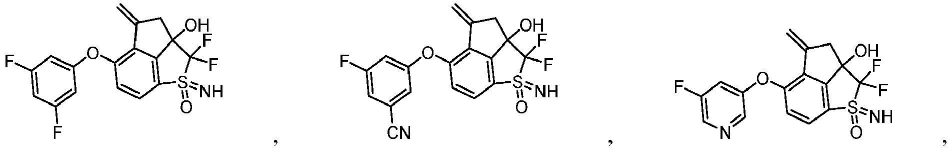

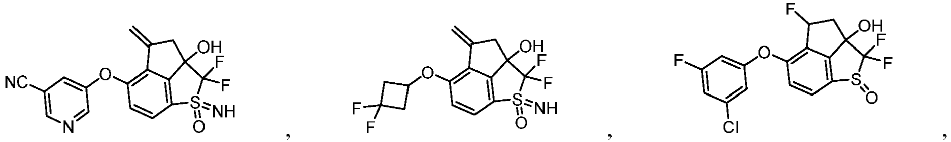

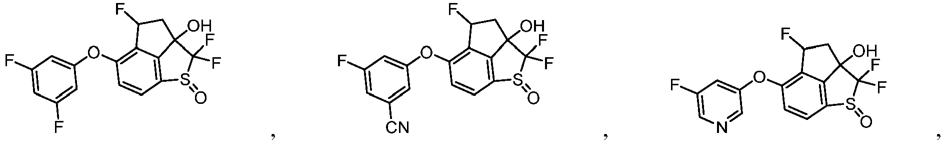

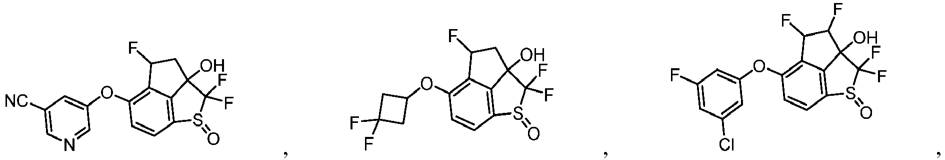

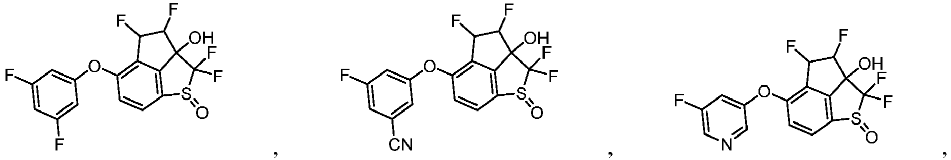

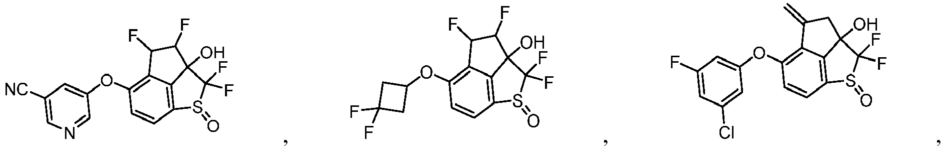

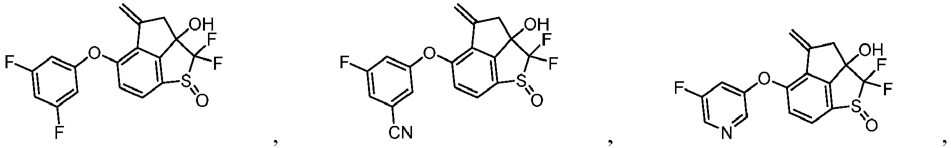

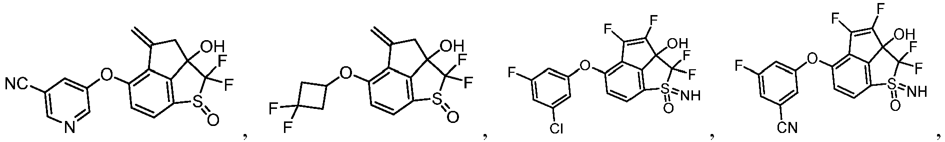

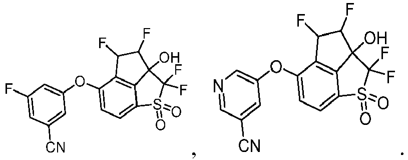

The present disclosure also provides a compound of the following formula, an optical isomer thereof, or a pharmaceutically acceptable salt thereof, selected from:

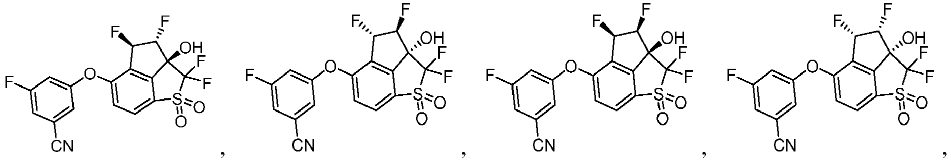

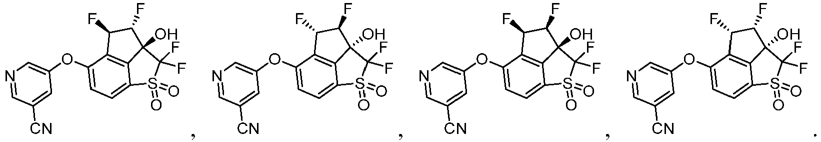

The present disclosure also provides a use of the compound or the pharmaceutically acceptable salt thereof or the pharmaceutical composition in the manufacture of a medicament for the prevention or treatment of HIF2α-mediated diseases.

In some embodiments of the present disclosure, the HIF2α-mediated diseases comprise renal cancer, brain glioma, Von Hippel-Lindau (VHL) syndrome, lung cancer, colorectal cancer, ovarian cancer, breast cancer, cervical cancer, gastric cancer, liver cancer, thyroid cancer, multiple myeloma, pancreatic ductal carcinoma, lung squamous cell carcinoma, colon cancer, hemangioma, pulmonary hypertension, and inflammatory bowel disease (IBD).

### Definition and description

Unless otherwise specified, the following terms and phrases when used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt comprises a salt of sodium, potassium, calcium, ammonium, organic amine, magnesium, or similar salts. When the compound of the present disclosure contains a relatively basic functional group, an acid addition salt can be obtained by bringing the neutral form of the compound into contact with a sufficient amount of acid in a solution or a suitable inert solvent. Examples of the pharmaceutically acceptable acid addition salt comprise an inorganic acid salt, wherein the inorganic acid comprises, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid comprises, for example, acetic acid, propionic acid, isobutyric acid, trifluoroacetic acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by a conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (R)- and (S)-enantiomers, diastereomers isomers, (D)-isomers, (L)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are comprised within the scope of the present disclosure.

The compounds of the present disclosure may exist in specific. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) comprises interconversion through proton migration, such as keto-enol isomerization and imine-enamine isomerization. Valence tautomer comprises some recombination of bonding electrons for mutual transformation. A specific example of keto-enol tautomerization is the tautomerism between two tautomers of pentane-2,4-dione and 4-hydroxypent-3-en-2-one.

The compound of the present disclosure may contain an unnatural proportion of atomic isotope at one or more than one atom that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs and the like. All isotopic variations of the compound of the present disclosure, whether radioactive or not, are encompassed within the scope of the present disclosure. "Optional" or "optionally" means that the subsequent event or condition may occur but not requisite, and the description includes the instance in which the event or condition occurs and the instance in which the event or condition does not occur.

The term "substituted by..." means one or more hydrogen atoms on a specific atom are substituted by the substituent, including deuterium and hydrogen variables, as long as the valence of the specific atom is normal and the substituted compound is stable. The term "optionally substituted by..." means an atom may or may not be substituted, unless otherwise specified, the type and number of the substituent may be arbitrary as long as being chemically achievable.

The term "absent" means that there is no substitution there, for example, when T₂ is absent, 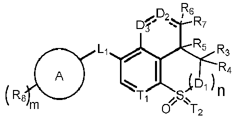 is

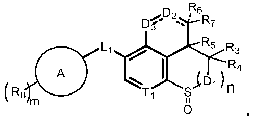

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound. For example, 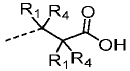 can be selected from 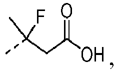 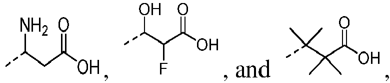 etc.

A hyphen ("-") being not between two letters or symbols indicates the linkage site of a substituent. For example, C₁₋₆ alkylcarbonyl- refers to a C₁₋₆ alkyl linked to the rest of the molecule through a carbonyl group. However, when the linkage site of a substituent is obvious to those skilled in the art, for example, a halogen substituent, "-" can be omitted.

Unless otherwise specified, when the valence bond of a group has a dashed line " 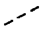 ", such as in 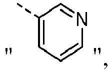 the dashed line indicates the linkage site of the group to the rest of the molecule. For example, in 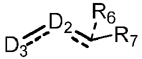 of the present disclosure, the group valence bond " 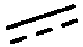 " represents a double bond " 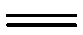 " or a single bond "-", and 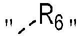 represents that R₆ 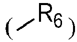 can be present or absent.

When one of the variables is selected from a single bond, it means that the two groups linked by the single bond are linked directly. For example, when D₃ in 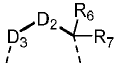 represents a single bond, the structure is actually

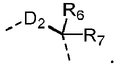

When the listed substituents do not indicate via which atom it is linked to the substituted group, this substituent can be bonded via any atom, for example, pyridyl, as a substituent, can link to the substituted group via any carbon atom on the pyridine ring.

When the listed linking group does not indicate the direction for linking, the direction for linking is arbitrary, for example, the linking group L contained in 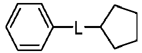 is 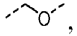 then 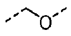 can link phenyl and cyclopentyl to form 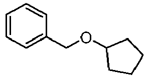 in the direction same as left-to-right reading order, and can link phenyl and cyclopentyl to form 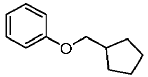 in the direction contrary to left-to-right reading order. A combination of the linking groups, substituents and/or variables thereof is allowed only when such combination can result in a stable compound.

Unless otherwise specified, the number of atoms on a ring is usually defined as the number of membered ring, such as a "4- to 6-membered ring" is a "ring" with 4 to 6 atoms arranged around it.

Unless otherwise specified, the term "C₁₋₆ alkyl" refers to a linear or branched saturated hydrocarbon group consisting of 1 to 6 carbon atoms. The C₁₋₆ alkyl includes C₁₋₅ alkyl, C₁₋₄ alkyl, C₂₋₆ alkyl, etc.; it can be monovalent (such as methyl), divalent (such as methylene), or multivalent (such as methine). Examples of C₁₋₅ alkyl include, but are not limited to, methyl ("Me"), ethyl ("Et"), propyl such as n-propyl ("n-Pr") or isopropyl ("i-Pr"), butyl such as n-butyl ("n-Bu"), isobutyl ("i-Bu"), sec-butyl ("s-Bu"), or tert-butyl ("t-Bu"), pentyl, hexyl, etc.

Unless otherwise specified, the term "C₁₋₃ alkyl" refers to a linear or branched saturated hydrocarbon group consisting of 1 to 3 carbon atoms. The C₁₋₃ alkyl includes C₁₋₂ alkyl, C₂₋₃ alkyl, etc.; it can be monovalent (such as methyl), divalent (such as methylene), or multivalent (such as methine). Examples of C₁₋₃ alkyl include, but are not limited to, methyl (Me), ethyl (Et), propyl (including *n*-propyl and isopropyl), etc.

Unless otherwise specified, the term "C₁₋₆ alkoxy" refers to an alkyl group containing 1 to 6 carbon atoms that are connected to the rest of the molecule through an oxygen atom. The C₁₋₆ alkoxy includes C₁₋₄ alkoxy, C₁₋₃ alkoxy, C₁₋₂ alkoxy, C₂₋₆ alkoxy, C₂₋₄ alkoxy, C₆ alkoxy, C₅ alkoxy, C₄ alkoxy, C₃ alkoxy, etc. Examples of C₁₋₆ alkoxy include, but are not limited to, methoxy, ethoxy, propoxy (including n-propoxy and isopropoxy), butoxy (including *n*-butoxy, isobutoxy, *s*-butoxy, and *t*-butoxy), pentyloxy (including *n*-pentyloxy, isopentyloxy, and neopentyloxy), hexyloxy, etc.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₂ alkoxy, C₂₋₃ alkoxy, C₃ alkoxy, C₂ alkoxy, etc. Examples of C₁₋₃ alkoxy include, but are not limited to, methoxy, ethoxy, propoxy (including n-propoxy and isopropoxy), etc.

Unless otherwise specified, "C₂₋₆ alkenyl" refers to a linear or branched hydrocarbon group consisting of 2 to 6 carbon atoms containing at least one carbon-carbon double bond, and the carbon-carbon double bond may be located at any position of the group. The C₂₋₆ alkenyl includes C₂₋₄ alkenyl, C₂₋₃ alkenyl, C₄ alkenyl, C₃ alkenyl, C₂ alkenyl, etc; it can be monovalent, divalent, or multivalent. Examples of C₂₋₆ alkenyl include, but are not limited to, vinyl, propenyl, butenyl, pentenyl, hexenyl, butadienyl, pentadienyl, hexadienyl, etc.

Unless otherwise specified, "C₂₋₃ alkenyl" refers to a linear or branched hydrocarbon group consisting of 2 to 3 carbon atoms containing at least one carbon-carbon double bond, and the carbon-carbon double bond may be located at any position of the group. The C₂₋₃ alkenyl includes C₃ alkenyl and C₂ alkenyl; it can be monovalent, divalent, or multivalent. Examples of C₂₋₃ alkenyl include, but are not limited to, vinyl, propenyl, etc.

Unless otherwise specified, "C₄₋₆ cycloalkyl" refers to a saturated cyclic hydrocarbon group consisting of 4 to 6 carbon atoms in monocyclic and bicyclic systems, and the C₄₋₆ cycloalkyl includes C₄₋₅ cycloalkyl, C₅₋₆ cycloalkyl, C₄ cycloalkyl, C₅ cycloalkyl, C₆ cycloalkyl, etc; it may be monovalent, divalent, or multivalent. Examples of C₄₋₆ cycloalkyl include, but are not limited to, cyclobutyl, cyclopentyl, cyclohexyl, etc.

Unless otherwise specified, the term "4- to 6-membered heterocycloalkyl" by itself or in combination with other terms refers to a saturated cyclic group consisting of 4 to 6 ring atoms, respectively, wherein 1, 2, 3, or 4 ring atoms are heteroatoms independently selected from O, S, and N and the rest are carbon atoms, wherein the nitrogen atoms are optionally quaternized, and the nitrogen and sulfur heteroatoms are optionally oxidized (i.e., NO and S(O)ₚ, p is 1 or 2). It includes monocyclic and bicyclic systems, wherein the bicyclic system includes spiro rings, fused rings, and bridged rings. In addition, in the case of the "4- to 6-membered heterocycloalkyl", the heteroatom may occupy the position where the heterocycloalkyl is linked to the rest of the molecule. The 4- to 6-membered heterocycloalkyl includes 5- to 6-membered heterocycloalkyl, 4-membered heterocycloalkyl, 5-membered heterocycloalkyl, 6-membered heterocycloalkyl, etc. Examples of 4- to 6-membered heterocycloalkyl include, but are not limited to, azetidinyl, oxetanyl, thietanyl, pyrrolidinyl, pyrazolidinyl, imidazolidinyl, tetrahydrothiophenyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxolyl, dithianyl, isoxazolidinyl, isothiazolidinyl, 1,2-oxazinyl, 1,2-thiazinyl, hexahydropyridazinyl, homopiperazinyl, homopiperidinyl, etc.

Unless otherwise specified, the term "5- to 6-membered heterocyclyl" by itself or in combination with other terms refers to a saturated or unsaturated cyclic group consisting of 4 to 6 ring atoms, respectively, wherein 1, 2, 3, or 4 ring atoms are heteroatoms independently selected from O, S, and N and the rest are carbon atoms, wherein the nitrogen atoms are optionally quaternized, and the nitrogen and sulfur heteroatoms are optionally oxidized (i.e., NO and S(O)ₚ, p is 1 or 2). It includes monocyclic and bicyclic systems, wherein the bicyclic system includes spiro rings, fused rings, and bridged rings. Examples of the "5- to 6-membered heterocyclyl" include, but are not limited to, cyclopentyl, cyclopentenyl, cyclohexyl, cyclohexenyl, oxocyclohexyl, tetrahydro-2H-pyran-2-keto, piperidin-2-keto, tetrahydro-2H-pyranyl, piperidinyl, etc.

Unless otherwise specified, the terms "5- to 6-membered heteroaromatic ring" and "5-to 6-membered heteroaryl" in the present disclosure may be used interchangeably, and the term "5- to 6-membered heteroaryl" refers to a monocyclic group consisting of 5 to 6 ring atoms with conjugated π electronic system, wherein 1, 2, 3, or 4 ring atoms are heteroatoms independently selected from O, S, and N, and the rest are carbon atoms. Where the nitrogen atom is optionally quaternized, and the nitrogen and sulfur heteroatoms are optionally oxidized (i.e., NO and S(O)ₚ, p is 1 or 2). The 5- to 6-membered heteroaryl may be attached to the rest of the molecule through a heteroatom or a carbon atom. The 5- to 6-membered heteroaryl includes 5-membered and 6-membered heteroaryl. Examples of the 5- to 6-membered heteroaryl include, but are not limited to, pyrrolyl (including N-pyrrolyl, 2-pyrrolyl, 3-pyrrolyl, etc.), pyrazolyl (including 2-pyrazolyl, 3-pyrazolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1*H*-1,2,3-triazolyl, 2*H*-1,2,3-triazolyl, 1*H*-1,2,4-triazolyl, 4*H*-1,2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridinyl (including 2-pyridyl, 3-pyridyl, 4-pyridyl, etc.), pyrazinyl, or pyrimidinyl (including 2-pyrimidinyl, 4-pyrimidinyl, etc.).

Unless otherwise specified, the term "cycloalkenyl" in the present disclosure refers to a cyclic alkenyl. "C₅₋₆ cycloalkenyl" includes C₅ cycloalkenyl, C₆ cycloalkenyl. Examples of cycloalkenyl include, but are not limited to, cyclopentenyl and cyclohexenyl.

Unless otherwise specified, Cₙ₋ₙ₊ₘ or Cₙ-Cₙ₊ₘ includes any specific instance of n to n+m carbons, for example, C₁₋₁₂ includes C₁, C₂, C₃, C₄, C₅, C₆, C₇, C₈, C₉, C₁₀, C₁₁, and C₁₂, also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, etc.; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, also includes any range from n to n+m, for example, 3- to 12-membered ring includes 3-to 6-membered ring, 3- to 9-membered ring, 5- to 6-membered ring, 5- to 7-membered ring, 6-to 7-membered ring, 6- to 8-membered ring, 6- to 10-membered ring, etc.

It will be understood by those skilled in the art that some compounds of formula (I) can contain one or more than one chiral center. Therefore, the compound has two or more stereoisomers. Therefore, the compounds of the present disclosure can be present in the form of individual stereoisomer (e.g., enantiomers, diastereomers) and mixtures thereof in arbitrary proportions, such as racemates, and under the proper condition, they can be present in the form of the tautomers and geometric isomers thereof.

As used herein, the term "stereoisomer" refers to compounds that have the same chemical constitution, but differ in the arrangement of the atoms or groups in space. Stereoisomer includes enantiomer, diastereomer, conformer, etc.

As used herein, the term "enantiomer" refers to two stereoisomers of a compound that are non-superimposable mirror images of each other.

As used herein, the term "diastereomer" refers to a stereoisomer in which a molecule has two or more chiral centers and whose molecules are not mirror images of each other. Diastereomers have different physical properties such as melting points, boiling points, spectral properties, or biological activities. Diastereomeric mixtures can be separated by high resolution analytical methods such as electrophoresis and chromatography (such as HPLC separation).

Stereochemical definitions and conventions can be followed in S. P. Parker ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. Many organic compounds are present in optically active forms, i.e., they have the ability to rotate the plane of plane polarized light. When optically active compounds are described, the prefixes D and L or R and S are used to denote the absolute configuration of the molecule with regard to its chiral centers. The prefixes d and 1 or (+) and (-) are used to denote the symbols of the compound's rotationally planar polarized light, wherein (-) or 1 indicates that the compound is levorotatory. Compounds with a prefix of (+) or d are dextrorotatory. For a given chemical structure, these stereoisomers are identical except that they are mirror images of each other. Specific stereoisomers can also be referred to as enantiomers, and mixtures of such isomers are often referred to as enantiomeric mixtures. A mixture of enantiomers in a ratio of 50 to 50 is known as a racemic mixture or racemate, which can be present in a chemical reaction or process without stereoselectivity or stereospecificity. The terms "racemic mixture" and "racemate" refer to an equimolar mixture of two enantiomers that is not optically active.

Racemic mixture can be used in its own form or after it is resolved into individual isomers. Resolution may yield stereochemically pure compounds or a mixture enriched in one or more isomers. Methods for separating isomers are well known (see Allinger N. L. and Eliel E. L., "Topics in Stereochemistry", Vol. 6, Wiley Interscience, 1971), including physical methods such as chromatography using chiral adsorbents. Individual isomers in a chiral form can be prepared from chiral precursors. Alternatively, a diastereomer salt can be formed with a chiral acid (such as a single enantiomer of 10-camphorsulfonic acid, camphoric acid, α-bromocamphoric acid, tartaric acid, diacetyltartaric acid, malic acid, pyrrolidone-5-carboxylic acid), and then the mixture was chemically separated to obtain a single isomer. The salt is then graded and crystallized, and one or both of the split bases are freed. This process can be optionally repeated to obtain one or two isomers that essentially do not contain the other isomer, i.e., the desired stereoisomer with an optical purity of at least 91%, 92%, 93%, 94%, 95%, 96%, 97%, 98%, 99%, or 99.5% by weight. Alternatively, the racemate can be covalently linked to a chiral compound (auxiliary) to obtain diastereomers, as is well known to those skilled in the art.

The term "tautomer" or "tautomeric form" as used herein refers to structural isomers of different energies that are interconvertible via a low energy barrier. For example, proton tautomers (also called prototropic tautomers) include interconversion through proton migration, such as keto-enol isomerization and imine-enamine isomerization. Valence tautomers include interconversions by recombination of some bonding electrons.

The term "treatment" as used herein refers to the administration of one or more pharmaceutical substances, in particular compounds of formula (I) and/or pharmaceutically acceptable salts thereof, to an individual suffering from a disease or having symptoms of the disease, for the purpose of curing, alleviating, mitigating, modifying, healing, improving, ameliorating or affecting the disease or symptoms of the disease. As used herein, the term "prevention" refers to the administration of one or more pharmaceutical substances, especially the compound of formula (I) described herein and/or the pharmaceutically acceptable salt thereof, to an individual with a constitution susceptible to the disease, to prevent the individual from suffering from the disease. When referring to chemical reactions, the terms "treating", "contacting", and "reacting" refer to adding or mixing two or more reagents under appropriate conditions to produce the indicated and/or desired products. It should be understood that the reaction to produce the indicated and/or desired products may not necessarily come directly from the combination of the two reagents initially added, i.e., there may be one or more intermediates generated in the mixture, which eventually lead to the formation of the indicated and/or desired products.

As used herein, the term "effective amount" refers to an amount generally sufficient to produce a beneficial effect on an individual. The effective amount of a compound of the present disclosure can be determined by conventional methods (such as modeling, dose-escalation studies, or clinical trials) in combination with conventional influencing factors (such as mode of administration, pharmacokinetics of the compound, severity and duration of the disease, medical history of the individual, health status of the individual, degree of response of the individual to the drug, etc.).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The technical and scientific terms used herein that are not specifically defined have the meanings commonly understood by those skilled in the art to which the present disclosure belongs.

### DETAILED DESCRIPTION OF THE PREFERRED EMBODIMENT

The present disclosure is further described in detail by the examples below. But it should be understood that these examples are only used to illustrate the present disclosure and are not intended to limit the scope of the present disclosure. Experimental methods in the following examples in which specific conditions are not indicated are usually in accordance with the conventional conditions for this type of reaction, or in accordance with the conditions recommended by the manufacturer. Unless otherwise specified, percentages and parts are by weight. Unless otherwise specified, ratios of liquids are by volume.

The experimental materials and reagents used in the following examples can be obtained from commercially available sources unless otherwise specified.

The following abbreviations are used in the present disclosure: DAST represents diethylaminosulfur trifluoride; DCM represents dichloromethane; DCE represents 1,2-dichloroethane; DMF represents N,N-dimethylformamide; Oxone represents potassium peroxomonosulfate; Selectfluor represents 1-chloromethyl-4-fluoro-1,4-diazoniabicyclo[2.2.2]octane bis(tetrafluoroborate); Pd₂(dba)₃ represents tris(dibenzylideneacetone)dipalladium.

### Example 1 Synthesis of compound 1

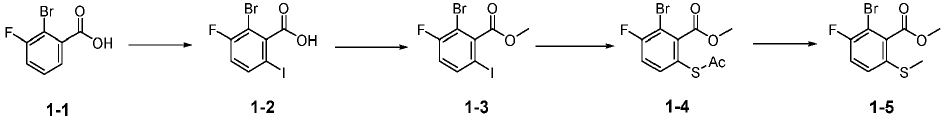

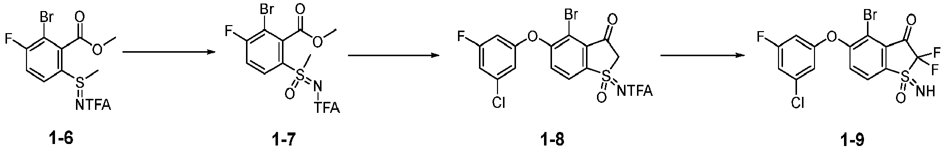

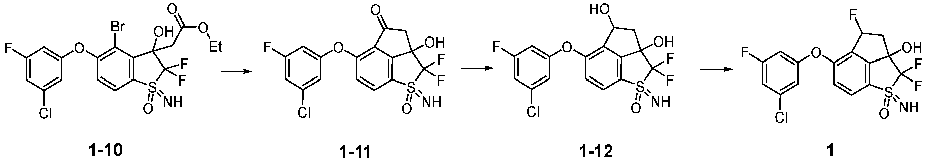

### Step 1: Preparation of compound 1-2

To a mixture of compound 1-1 (2.5 g, 11.4 mmol), palladium acetate (128 mg, 0.57 mmol), iodine (2.9 g, 11.4 mmol), and (diacetoxyiodo)benzene (3.68 g, 11.4 mmol) was added DMF (55 mL). The reaction mixture was replaced with argon three times, and stirred at 100°C for 24 hours. The reaction mixture was cooled to room temperature, concentrated under reduced pressure to remove most of the DMF. The crude product was poured into dilute hydrochloric acid (100 mL, 0.1 M), and the mixture was extracted three times with 400 mL of ethyl acetate. The organic phases were combined, washed with 1M sodium thiosulfate, then washed with saturated brine, and dried over anhydrous sodium sulfate. After filtration, the filtrate was concentrated to obtain compound **1-2,** and the crude product was directly used in the next step.

### Step 2: Preparation of compound 1-3

Compound **1-2** (0.81 g, 2.3 mmol) was dissolved in DMF (5 mL), and potassium carbonate (970 mg, 7.0 mmol) and iodomethane (0.44 mL, 7.0 mmol) were added thereto. The reaction mixture was stirred at room temperature for 18 hours. The reaction mixture was added with water (30 mL) and extracted twice with 60 mL of ethyl acetate. The combined organic phase was washed five times with water and then with saturated brine, dried over anhydrous sodium sulfate, and filtered. The filtrate was concentrated to obtain compound **1-3.** The crude product was used directly in the next step. LCMS m/z = 358.9/360.9 [M+1]⁺.

### Step 3: Preparation of compound 1-4

Compound **1-3** (1.26 g, 3.5 mmol) and 4,5-bis(diphenylphosphino)-9,9-dimethylxanthene (Xantphos, 243 mg, 0.42 mmol) were dispersed in toluene: acetone (17 mL, v/v = 2:1). Pd₂(dba)₃ (192 mg, 0.21 mmol) and potassium thioacetate (500 mg, 4.4 mmol) were added thereto. The reaction mixture was replaced with argon, sealed, heated to 70°C and stirred for 2 hours. The reaction mixture was cooled to room temperature, diluted with dichloromethane and filtered, and the filter cake was washed twice with dichloromethane. The filtrates were combined and dried over anhydrous sodium sulfate. After filtration, the filtrate was concentrated and subjected to column chromatography to obtain compound **1-4.** ¹H NMR (400 MHz, CDCl₃) δ = 7.44-7.41 (m, 1H), 7.25-7.21 (m, 1H), 3.95 (s, 3H), 2.42 (s, 3H); LCMS m/z = 306.9/308.9 [M+1]⁺.

### Step 4: Preparation of compound 1-5

Compound **1-4** (1.21 g, 3.9 mmol) was dissolved in 12 mL of methanol, and cesium carbonate (1.66 g, 5.1 mmol) was added thereto after the system was replaced with argon. The reaction mixture was stirred at room temperature for 1 hour, added with iodomethane (1.22 mL, 20 mmol), and then continued to stir for 16 hours. The reaction mixture was concentrated under reduced pressure, dispersed in 30 mL of water, and extracted three times with 90 mL of ethyl acetate. The combined organic phase was washed with saturated brine, dried over anhydrous sodium sulfate, filtered, and the filtrate was concentrated and then subjected to column chromatography to obtain compound **1-5.** ¹H NMR (400 MHz, CDCl₃) δ = 7.38-7.35 (m, 1H), 7.16-7.11 (m, 1H), 3.99 (s, 3H), 2.45 (s, 3H); LCMS m/z = 247.0/249.0 [M+1-MeOH]⁺.

### Step 5: Preparation of compound 1-6

To a mixture of compound **1-5** (5.9 g, 21.1 mmol), trifluoroacetamide (11.9 g, 106 mmol), magnesium oxide (11.9 g, 296 mmol), and (diacetoxyiodo)benzene (35.4 g, 110 mmol) was added dichloromethane (150 mL) and rhodium(II) octanoate dimer (140 mg, 0.2 mmol). The reaction mixture was replaced with argon and stirred at 40°C for 16 hours. The reaction mixture was cooled to room temperature, diluted with dichloromethane and filtered, and the filter cake was washed twice with dichloromethane. The filtrates were combined and dried over anhydrous sodium sulfate. After filtration, the filtrate was concentrated and subjected to column chromatography to obtain compound **1-6.** ¹H NMR (400 MHz, CDCl₃) δ = 8.07-8.04 (m, 1H), 7.49-7.45 (m, 1H), 4.07 (s, 3H), 3.05 (s, 3H).

### Step 6: Preparation of compound 1-7

To a solution of compound **1-6** (5.0 g, 12.8 mmol) in carbon tetrachloride/acetonitrile (60 mL, 1:1) was added a solution of sodium periodate (8.22 g, 38.4 mmol) in water (15 mL) and ruthenium(III) chloride (80 mg, 0.38 mmol), and the reaction mixture was stirred at room temperature for 16 hours. The reaction mixture was concentrated under reduced pressure to remove carbon tetrachloride and acetonitrile. The remaining aqueous phase was added with water (50 mL) and extracted three times with 210 mL of ethyl acetate. The combined organic phase was washed with saturated brine, dried over anhydrous sodium sulfate, filtered, and the filtrate was concentrated and then subjected to column chromatography to obtain compound **1-7.** ¹H NMR (400 MHz, CDCl₃) δ = 8.17-8.14 (m, 1H), 7.47-7.43 (m, 1H), 4.01 (s, 3H), 3.61 (s, 3H); LCMS m/z = 406.0/408.0 [M+1]⁺.

### Step 7: Preparation of compound 1-8

To DMF (10 mL) was added compound **1-7** (1.0 g, 2.46 mmol), 3-chloro-5-fluorophenol (1.08 g, 7.39 mmol), and potassium carbonate (510 mg, 3.69 mmol), respectively. The reaction mixture was heated to 130°C and reacted for 10 minutes under microwave irradiation. The reaction mixture was cooled to room temperature, added with 30 mL of water, and extracted three times with 100 mL of ethyl acetate. The combined organic phase was washed with water three times, then washed with saturated brine, dried over anhydrous sodium sulfate, and filtered. The filtrate was concentrated and subjected to column chromatography to obtain the compound **1-8.** ¹H NMR (400 MHz, CDCl₃) δ = 8.30 (d, *J* = 8.4 Hz, 1H), 7.43 (d, *J* = 8.4 Hz, 1H), 7.07-7.04 (m, 1H), 6.90-6.88 (m, 1H), 6.74 (dt, *J =* 8.8, 2.4 Hz, 1H), 4.69 (d, *J* = 17.6 Hz, 1H), 4.41 (d, *J* = 17.6 Hz, 1H); LCMS m/z = 500.0/501.9 [M+1]⁺.

### Step 8: Preparation of compound 1-9

To 20 mL of acetonitrile was added compound **1-8** (350 mg, 4.26 mmol), and then Na₂CO₃ (222 mg, 2.1 mmol) was added thereto. Under argon atmosphere, the reaction mixture was stirred at room temperature for 10 minutes, and Selectfluor (743 mg, 2.1 mmol) was added thereto. The reaction mixture was stirred at room temperature for 4 hours. The reaction mixture was concentrated under reduced pressure, added with 50 mL of water, extracted four times with 120 mL of ethyl acetate. The combined organic phase was washed with saturated brine, and dried over anhydrous sodium sulfate. Anhydrous sodium sulfate was filtered off, and the filtrate was concentrated and subjected to column chromatography to obtain compound **1-9.** ¹H NMR (400 MHz, CDCl₃) δ = 8.08 (d, *J* = 8.4 Hz, 1H), 7.48 (d, *J* = 8.4 Hz, 1H), 7.05-7.02 (m, 1H), 6.86-6.84 (m, 1H), 6.72 (dt, *J* = 8.8, 2.4 Hz, 1H), 5.30 (s, 1H); LCMS m/z = 439.8/401.8 [M+1]⁺.

### Step 9: Preparation of compound 1-10

Ethyl acetate (180 mg, 2.04 mmol) was dissolved in tetrahydrofuran (4 mL). The mixture was replaced with argon three times, cooled to -70°C, then added slowly with LDA (1.02 mL, 2.04 mmol, 2M tetrahydrofuran solution), and stirred for 30 minutes. Then a solution of compound **1-9** (300 mg, 0.68 mmol) in tetrahydrofuran (3 mL) was added slowly thereto. The reaction mixture was stirred at -70°C for 1 hour. The reaction mixture was quenched with 10 mL of saturated ammonium chloride solution, warmed to room temperature, and extracted four times with 40 mL of dichloromethane. The combined organic phase was washed with saturated brine, and dried over anhydrous sodium sulfate. Anhydrous sodium sulfate was filtered off, and the filtrate was concentrated and subjected to column chromatography to obtain compound **1-10.** LCMS m/z = 528.0/530.0 [M+1]⁺.

### Step 10: Preparation of compound 1-11

Compound **1-10** (100 mg, 0.19 mmol) was dissolved in tetrahydrofuran (2 mL). The mixture was replaced with argon three times, cooled to -70°C, added slowly with n-butyllithium (0.38 mL, 0.95 mmol, 2.5 M n-hexane solution). The reaction mixture was stirred at -70°C for 30 minutes after the addition was completed. The reaction mixture was quenched with dilute hydrochloric acid (0.5 mL, 0.5 M), warmed to room temperature, added with 10 mL of water, and extracted four times with 40 mL of dichloromethane. The combined organic phase was washed with saturated brine and dried over anhydrous sodium sulfate. Anhydrous sodium sulfate was filtered off, and the filtrate was concentrated and subjected to column chromatography to obtain compound **1-11.** ¹H NMR (400 MHz, CDCl₃) δ = 7.99-7.93 (m, 1H), 7.11-7.05 (m, 2H), 6.98-6.97 (m, 1H), 6.83-6.80 (m, 1H), 3.26-3.20 (m, 1H), 3.07-3.01 (m, 1H); LCMS m/z = 404.0/406.0 [M+1]⁺.

### Step 11: Preparation of compound 1-12

Compound **1-11** (50 mg, 0.12 mmol) was dissolved in ethanol (2 mL). The reaction mixture was cooled to -70°C, added with sodium borohydride (7 mg, 0.19 mmol), and stirred for 15 minutes at -70°C after the addition was completed. The reaction mixture was quenched with dilute hydrochloric acid (0.5 mL, 0.5 M), warmed to room temperature, added with 3 mL of water, and extracted four times with 12 mL of dichloromethane. The combined organic phase was washed with saturated brine and dried over anhydrous sodium sulfate. Anhydrous sodium sulfate was filtered off, and the filtrate was concentrated and subjected to preparative HPLC to obtain compound **1-12.**

¹H NMR (400 MHz, CDCl₃) δ = 7.73 (d, *J* = 8.4 Hz, 1H), 7.10 (dd, *J* = 8.4, 1.6 Hz, 1H), 7.00 (dt, *J* = 8.0, 2.0 Hz, 1H), 6.93-6.92 (m, 1H), 6.76 (dt, *J* = 8.4, 2.0 Hz, 1H), 5.94 (t, *J* = 6.8 Hz, 1H), 3.11 (brs, 1H), 3.01-2.89 (m, 1H), 2.53-2.48 (m, 1H), 2.33 (brs, 1H); LCMS m/z = 406.0/408.0 [M+1]⁺.

### Step 12: Preparation of compound 1

Compound **1-12** (80 mg, 0.20 mmol) was dissolved in DCE (2 mL). The reaction mixture was cooled to 0°C, added with DAST (38 mg, 0.24 mmol), and stirred for 1 hour at 0°C after the addition was completed. The reaction mixture was quenched with water (3 mL), warmed to room temperature, and extracted four times with 12 mL of dichloromethane. The combined organic phase was washed with saturated brine, and dried over anhydrous sodium sulfate. Anhydrous sodium sulfate was filtered off, and the filtrate was concentrated and subjected to preparative HPLC to obtain compound **1.**

¹H NMR (400 MHz, CDCl₃) δ = 7.79 (dd, *J* = 8.4, 2.0 Hz, 1H), 7.11 (dd, *J* = 8.4, 1.2 Hz, 1H), 7.03 (dt, *J* = 8.0, 2.0 Hz, 1H), 6.96-6.95 (m, 1H), 6.79 (dt, *J* = 8.8, 2.4 Hz, 1H), 6.03 (dd, *J =* 52.8, 5.2 Hz, 1H), 3.75 (brs, 1H), 2.96-2.64 (m, 2H); LCMS m/z = 408.0/410.0 [M+1]⁺.

### Example 2: Synthesis of compound 2

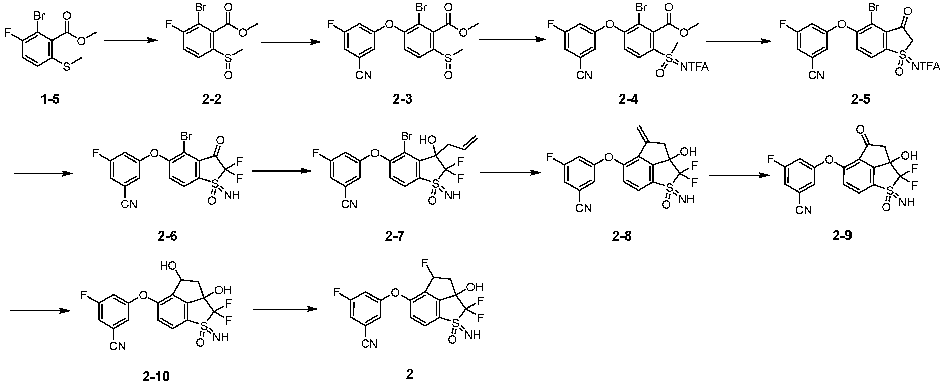

### Step 1: Preparation of compound 2-2

To a solution of compound **1-5** (15.0 g, 53.7 mmol) in methanol (110 mL) was added dropwise a cloudy solution of Oxone (16.52 g, 26.9 mmol) in water (55 mL) in an ice bath. After the addition was completed, the reaction mixture was naturally warmed to room temperature, and stirred at room temperature for 16 hours. The reaction mixture was concentrated under reduced pressure to remove most of the methanol, and then dispersed in ethyl acetate (200 mL) and water (200 mL). The aqueous phase was extracted three times with 300 mL ethyl acetate. The organic phases were combined, washed with 1 M sodium thiosulfate, then washed with saturated brine, and dried over anhydrous sodium sulfate. After filtration, the filtrate was concentrated, and the crude product was purified by a flash silica gel column to obtain compound **2-2.** LCMS m/z = 295.0/297.0 [M+1]⁺.

### Step 2: Preparation of compound 2-3

Compound **2-2** (7.0 g, 23.7 mmol) was dissolved in DMF (120 mL) at room temperature, and potassium carbonate (4.92 g, 35.6 mmol) and 3-cyano-5-fluorophenol (4.88 g, 35.6 mmol) were added thereto. The reaction mixture was replaced with argon and then stirred at 90°C for 8 hours. The reaction mixture was cooled to room temperature, concentrated under reduced pressure to remove most of the DMF. The crude product was dispersed in ethyl acetate (100 mL) and water (100 mL). The aqueous phase was extracted twice with 100 mL of ethyl acetate. The combined organic phase was washed with water, then washed with saturated brine, dried over anhydrous sodium sulfate, filtered, and the filtrate was concentrated and then the crude product was purified by a flash silica gel column to obtain compound **2-3.** ¹H NMR (400 MHz, CDCl₃) δ = 8.11 (d, *J* = 8.8 Hz, 1H), 7.35 (d, *J* = 8.8 Hz, 1H), 7.18 (ddd, *J* = 7.6, 2.4, 1.2 Hz, 1H), 7.03-7.02 (m, 1H), 6.96 (dt, *J* = 9.2, 2.4 Hz, 1H), 4.01 (s, 3H), 2.88 (s, 3H), LCMS m/z = 412.0/414.0 [M+1]⁺.

### Step 3: Preparation of compound 2-4

To a mixture of compound **2-3** (3.0 g, 7.28 mmol), trifluoroacetamide (2.88 g, 25.5 mmol), magnesium oxide (2.35 g, 58.2 mmol), and (diacetoxyiodo)benzene (8.2 g, 25.5 mmol) was added dichloromethane (30 mL) and rhodium(II) octanoate dimer (113 mg, 0.15 mmol). The reaction mixture was replaced with argon and stirred at 40°C for 16 hours. The reaction mixture was cooled to room temperature, diluted with dichloromethane and filtered, and the filter cake was washed twice with dichloromethane. The filtrates were combined and dried over anhydrous sodium sulfate. After filtration, the filtrate was concentrated and purified by column chromatography to obtain compound **2-4.** ¹H NMR (400 MHz, CDCl₃) δ = 8.12 (d, *J* = 8.8 Hz, 1H), 7.29 (ddd, *J* = 7.6, 2.4, 1.2 Hz, 1H), 7.19-7.15 (m, 2H), 7.07 (dt, *J* = 8.8, 2.4 Hz, 1H), 4.02 (s, 3H), 3.63 (s, 3H), LCMS m/z = 523.0/525.0 [M+1]⁺.

### Step 4: Preparation of compound 2-5

Compound **2-4** (2.03 g, 3.9 mmol) was dissolved in THF (20 mL), and the mixture was added with cesium carbonate (1.52 g, 4.7 mmol) and sealed, and the reaction mixture was stirred at 90°C for 1 hour. The reaction mixture was cooled to room temperature, added with 30 mL of water, and extracted three times with 90 mL of ethyl acetate. The combined organic phase was washed with saturated brine, dried over anhydrous sodium sulfate, and filtered. The filtrate was concentrated and purified by column chromatography to obtain compound **2-5.** ¹H NMR (400 MHz, CDCl₃) δ = 8.37 (d, *J* = 8.8 Hz, 1H), 7.49 (d, *J* = 8.8 Hz, 1H), 7.29 (ddd, *J* = 7.6, 2.4, 1.2 Hz, 1H), 7.13-7.12 (m, 1H), 7.05 (dt, *J* = 8.8, 2.4 Hz, 1H), 4.70 (d, *J* = 17.6 Hz, 1H), 4.43 (d, *J* = 17.6 Hz, 1H), LCMS m/z = 491.0/493.0 [M+1]⁺.

### Step 5: Preparation of compound 2-6

To 20 mL of acetonitrile was added compound **2-5** (1.00 g, 2.04 mmol), and then added Na₂CO₃ (647 mg, 6.1 mmol) and Selectfluor (2.16 g, 6.1 mmol). The reaction mixture was stirred at room temperature for 3 hours. The reaction mixture was concentrated under reduced pressure, added with 50 mL of water, extracted four times with 120 mL of ethyl acetate. The combined organic phase was washed with saturated brine and dried over anhydrous sodium sulfate. Anhydrous sodium sulfate was filtered off, and the filtrate was concentrated to obtain the crude product of compound **2-6.** LCMS m/z = 449.0/451.0 [M+H₂O+1]⁺.

### Step 6: Preparation of compound 2-7

Compound **2-6** (1.0 g, 2.32 mmol, crude product) was dissolved in DMF (20 mL), and the mixture was added with indium (532 mg, 4.64 mmol) and allyl iodide (1.17 g, 6.96 mmol), and the reaction mixture was stirred for 2 hours at room temperature. The reaction mixture was added with 1 M dilute hydrochloric acid (15 mL) and extracted three times with 30 mL of ethyl acetate. The combined organic phase was washed with saturated brine, dried over anhydrous sodium sulfate, filtered, and the filtrate was concentrated and then subjected to column chromatography to obtain compound **2-7.** LCMS m/z = 473.0/475.0 [M+1]⁺.

### Step 7: Preparation of compound 2-8

To compound **2-7** (380 mg, 0.80 mmol), triethylamine (0.33 mL, 2.41 mmol), trimesitylphosphine (31 mg, 0.08 mmol), and Pd₂(dba)₃ (74 mg, 0.08 mmol) were added DMF (8 mL). The reaction mixture was replaced with nitrogen, heated to 90°C, and reacted for 3 hours. The reaction mixture was cooled to room temperature, added with 30 mL of water, and extracted three times with 100 mL of ethyl acetate. The combined organic phase was washed with water three times, then washed with saturated brine, dried over anhydrous sodium sulfate, and filtered. The filtrate was concentrated and subjected to column chromatography to obtain compound **2-8.** LCMS m/z = 393.0 [M+1]⁺.

### Step 8: Preparation of compound 2-9

Compound **2-8** (100 mg, 0.25 mmol) was dissolved in acetonitrile (6 mL) and water (1 mL). The mixture was added with NaIO₄ (136 mg, 0.64 mmol) and RuCl₃ (5 mg, 0.02 mmol), stirred at room temperature for 2 hours, and then added with additional NaIO₄ (68 mg, 0.32 mmol) and RuCl₃ (3 mg, 0.01 mmol). The reaction mixture was continued to stir for 16 hours at room temperature. The reaction mixture was quenched with saturated sodium thiosulfate (3 mL), concentrated to remove most of the acetonitrile, and dispersed in 10 mL of ethyl acetate and 10 mL of water. The aqueous phase was extracted four times with 20 mL of ethyl acetate. The combined organic phase was washed with saturated brine and dried over anhydrous sodium sulfate. After filtration, the filtrate was concentrated to obtain the crude product of compound **2-9.** LCMS m/z = 395.0 [M+1]⁺.

### Step 9: Preparation of compound 2-10

Compound **2-9** (78 mg, 0.20 mmol) was dissolved in ethanol (1 mL). The reaction mixture was cooled to -70°C, added with sodium borohydride (14 mg, 0.38 mmol), and stirred for 15 minutes at -70°C after the addition was completed. The reaction mixture was quenched with dilute hydrochloric acid (0.5 mL, 0.5 M), warmed to room temperature, added with 3 mL of water, and extracted four times with 12 mL of dichloromethane. The combined organic phase was washed with saturated brine and dried over anhydrous sodium sulfate. After filtration, the filtrate was concentrated, and then the crude product was purified by a flash silica gel column to obtain compound **2-10.** LCMS m/z = 397.0 [M+1]⁺.

### Step 10: Preparation of compound 2

Compound **2-10** (80 mg, 0.20 mmol) was dissolved in DCM (5 mL). The reaction mixture was cooled to -70°C, added with DAST (39 mg, 0.24 mmol). After the addition was completed, the reaction mixture was stirred for 0.5 hours at -70°C. The reaction mixture was added with DAST (18 mg, 0.12 mmol). After the addition was completed, the reaction mixture was stirred at -70°C for 0.5 hours. The reaction mixture was quenched with methanol (1 mL) at -70°C, warmed to room temperature, concentrated and subjected to preparative SFC twice (the first time: column DAICELCHIRALPAK^{®}AS (250 * 25 mm, 10 µm); mobile phase [0.1% diethylamine, methanol]; B%: 20% to 20%. The second time: column DAICELCHIRALPAK^{®}IG (250 * 25 mm, 10 µm); mobile phase [0.1% diethylamine, methanol]; B%: 30% to 30) to obtain compound 2 with a retention time of 3.445 minutes. The retention time was determined with the following analytical column: column: Dr.maish Reprosil Chiral-MIC (DAICELCHIRALPAK^{®}IC) 100 * 3.0 mm 3 µm, mobile phase: A: carbon dioxide B: methanol (0.1% diethylamine), 40% B, flow rate: 1.5 mL/min, column temperature: 35°C.

Compound **2:** ¹H NMR (400 MHz, CD₃OD) δ = 7.89 (d, *J* = 8.4 Hz, 1H), 7.54-7.46 (m, 1H), 7.45-7.44 (m, 1H), 7.38 (dd, *J* = 9.2, 2.4 Hz, 1H), 7.29 (d, *J* = 8.4 Hz, 1H), 6.05 (dd, *J* = 53.2, 5.2 Hz, 1H), 2.93-2.80 (m, 1H), 2.65-2.58 (m, 1H), LCMS m/z = 399.0 [M+1]⁺.

### Example 3: Synthesis of compound 3-P1, compound 3-P2, compound 3-P3, and compound 3-P4

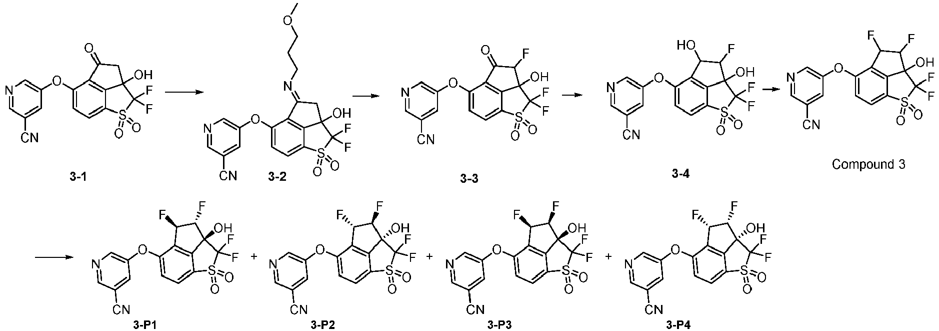

### Step 1: Preparation of compound 3-2

To toluene (10 mL) was added compound **3-1** (1.00 g, 2.64 mmol), 3-methoxypropylamine (470 mg, 5.28 mmol), p-toluenesulfonic acid (45.5 mg, 0.264 mmol), and magnesium sulfate (636 mg, 5.28 mmol). The reaction mixture was stirred at room temperature for 16 hours. LCMS showed the reaction was completed. The reaction mixture was concentrated under reduced pressure to obtain the crude product of compound **3-2** (2.11 g), which was directly used in the next step. LCMS m/z = 450.01 [M+1]⁺.

### Step 2: Preparation of compound 3-3

The crude product of compound **3-2** (1.00 g) was dissolved in acetonitrile (10 mL). The mixture was added with 1-chloromethyl-4-fluoro-1,4-diazoniabicyclo[2.2.2]octane bis(tetrafluoroborate) (1.57 g, 4.44 mmol), and anhydrous sodium carbonate (468 mg, 4.44 mmol), and continued to stir at room temperature for 2 hours. TLC showed the reaction was completed. The reaction mixture was adjusted to pH = 5 with 1 M HCl solution (100 mL) and stirred at room temperature for 1 hour. The reaction mixture was concentrated under reduced pressure, added with ethyl acetate (100 mL) for dissolution, washed successively with water (50 mL × 3) and saturated brine (50 mL × 3), dried over anhydrous sodium sulfate, filtered, and concentrated under reduced pressure to obtain a residue, which was subjected to column chromatography to obtain compound **3-3** (700 mg, two-step total yield of 70.8%). LCMS m/z = 397.30 [M+1]⁺. ¹H NMR (400 MHz, DMSO-d₆) δ = 9.05 (t, *J* = 7.0 Hz, 1H), 8.94 (d, *J* = 2.7 Hz, 1H), 8.63 (d, *J* = 8.6 Hz, 1H), 8.50 - 8.39 (m, 1H), 8.29 (s, 1H), 7.67 (d, *J* = 8.6 Hz, 1H), 5.87 (d, *J* = 48.7 Hz, 1H).

### Step 3: Preparation of compound 3-4

To a solution of compound **3-3** (700 mg, 1.77 mmol) in ethanol (10 mL) was added sodium borohydride (134 mg, 2.54 mmol) at -78°C, and the reaction mixture was reacted at this temperature for 1 hour. TLC showed the reaction was completed. The reaction mixture was poured into saturated ammonium chloride aqueous solution (50 mL), stirred for 10 minutes, and extracted with ethyl acetate (50 mL × 3). The combined organic phase was washed with saturated brine (50 mL × 3), dried over anhydrous sodium sulfate, and concentrated under reduced pressure, and the residue was purified by column chromatography to obtain compound **3-4** (540 mg, yield of 76.8%). ¹H NMR (400 MHz, DMSO-d₆) δ = 9.01 (d, *J* = 1.6 Hz, 1H), 8.95 (d, *J* = 3.2 Hz, 1H), 8.48 (dd, *J* = 2.7, 1.7 Hz, 1H), 8.32 (d, *J* = 2.0 Hz, 1H), 8.00 (s, 2H), 7.46 (d, *J* = 8.5 Hz, 1H), 6.21 (dd, *J* = 49.0, 13.5 Hz, 1H), 5.87 (d, *J* = 3.3 Hz, 1H).

### Step 4: Preparation of compound 3

To a solution of compound **3-4** (550 mg, 1.38 mmol) in 2-methyltetrahydrofuran (5 mL) was added dropwise a solution of diethylaminosulfur trifluoride (445 mg, 2.76 mmol) in 2-methyltetrahydrofuran (5 mL) at 0°C. The reaction mixture was continued to stir for 1 hour at this temperature. TLC showed the reaction was completed. The reaction mixture was poured into saturated sodium bicarbonate solution (50 mL) and extracted with ethyl acetate (50 mL × 2). The combined organic phase was washed with saturated brine (50 mL × 2), dried over anhydrous sodium sulfate, and concentrated under reduced pressure, and the residue was purified by column chromatography to obtain compound 3 (200 mg, yield of 36.2%). LCMS m/z = 401.01 [M+1]⁺.

### Step 5: Preparation of compounds 3-P1, 3-P2, 3-P3, and 3-P4

Compound 3 (450 mg, 1.12 mmol) was purified by preparative HPLC (FA system) to obtain a mixture of compound 3-P1 and compound 3-P2 (300 mg, the second eluent), a mixture of compound 3-P3 and compound 3-P4 (30 mg, the first eluent).

The mixture (300 mg) of compound 3-P1 and compound 3-P2 was subjected to SFC (column DAICELCHIRALPAK^{®}ID (250 * 25 mm, 10 µm)); mobile phase [A: carbon dioxide, B: methanol (containing 0.1% 7.0 mol/L ammonia water)]; B%: 0% to 30% to obtain compound 3-P1 (retention time of 2.495 minutes) and compound 3-P2 (retention time of 3.108 minutes). The retention time was determined by the following analytical method: column: DAICELCHIRALPAK^{®}IB, 250 * 25 mm, 10 µm, mobile phase [A: carbon dioxide, B: methanol (containing 0.1% 7.0 mol/L ammonia water)], 30% B, flow rate: 70 mL/ min, column temperature: 35°C. The mixture was separated by SFC to obtain the crude products of compound 3-P1 (80.0 mg, recovery rate of 17.8%) and compound 3-P2 (60.0 mg). The crude product of compound 3-P2 was purified by preparative HPLC (FA system) to obtain compound 3-P2 (49.1 mg, recovery rate of 10.9%). Compound 3-P1, ¹H NMR (400 MHz, DMSO-d₆) δ = 9.01 (d, *J* = 1.5 Hz, 1H), 8.95 (d, *J* = 2.7 Hz, 1H), 8.48 (dd, *J* = 2.7, 1.7 Hz, 1H), 8.31 (dd, *J* = 8.6, 2.0 Hz, 1H), 8.01 (s, 1H), 7.46 (d, *J* = 8.6 Hz, 1H), 6.21 (dd, *J* = 49.0, 13.5 Hz, 1H), 5.80 (dd, *J* = 46.9, 5.9 Hz, 1H). Compound 3-P2, ¹H NMR (400 MHz, DMSO-d₆) δ = 9.01 (d, *J* = 1.5 Hz, 1H), 8.95 (d, *J* = 2.7 Hz, 1H), 8.48 (dd, *J* = 2.7, 1.7 Hz, 1H), 8.31 (dd, *J* = 8.6, 2.0 Hz, 1H), 8.01 (s, 1H), 7.46 (d, *J* = 8.6 Hz, 1H), 6.21 (dd, *J* = 49.0, 13.5 Hz, 1H), 5.80 (dd, *J* = 46.9, 5.9 Hz, 1H).

The mixture (30 mg) of compound 3-P3 and compound 3-P4 was subjected to SFC (column DAICELCHIRALPAK^{®}IB (250 * 25 mm, 10 µm)); mobile phase [A: carbon dioxide, B: methanol (containing 0.1% 7.0 mol/L ammonia water)]; B%: 0% to 30% to obtain compound 3-P3 (retention time of 3.325 minutes) and compound 3-P4 (retention time of 3.544 minutes). The retention time was determined by the following analytical method: column: DAICELCHIRALPAK^{®}IB, 250 * 25 mm, 10 µm, mobile phase [A: carbon dioxide, B: methanol (containing 0.1% 7.0 mol/L ammonia water)], 30% B, flow rate: 70 mL/ min, column temperature: 35°C. The mixture was separated by SFC to obtain compound 3-P3 (4.44 mg, recovery rate of 0.99%) and compound 3-P4 (4.26 mg, recovery rate of 0.95%). Compound 3-P3, ¹H NMR (400 MHz, DMSO-d₆) δ = 9.00 (d, *J* = 1.6 Hz, 1H), 8.90 (d, *J* = 2.7 Hz, 1H), 8.39 (dd, *J* = 2.6, 1.7 Hz, 1H), 8.32 (dd, *J* = 8.6, 1.9 Hz, 1H), 7.93 (s, 1H), 7.51 (d, *J* = 8.6 Hz, 1H), 6.09 (dd, *J* = 54.6, 4.6 Hz, 1H), 5.39 (ddd, *J* = 46.7, 16.7, 4.4 Hz, 1H). Compound 3-P4, ¹H NMR (400 MHz, DMSO-d₆) δ = 9.00 (d, *J* = 1.6 Hz, 1H), 8.90 (d, *J* = 2.7 Hz, 1H), 8.39 (dd, *J* = 2.6, 1.7 Hz, 1H), 8.32 (dd, *J* = 8.6, 1.9 Hz, 1H), 7.93 (s, 1H), 7.51 (d, *J* = 8.6 Hz, 1H), 6.09 (dd, *J* = 54.6, 4.6 Hz, 1H), 5.39 (ddd, *J* = 46.7, 16.7, 4.4 Hz, 1H).

### Example 4: Synthesis of compound 4, compound 4-P1, compound 4-P2, compound 4-P3, and compound 4-P4

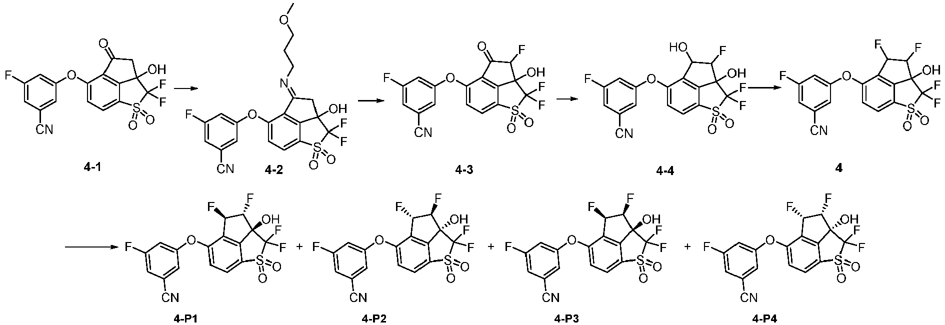

### Step 1: Preparation of compound 4-2

To toluene (50 mL) was added compound **4-1** (2.50 g, 6.32 mmol), 3-methoxypropylamine (3.70 g, 41.1 mmol), p-toluenesulfonic acid (109 mg, 0.632 mmol), and magnesium sulfate (1.50 g, 12.6 mmol). The reaction mixture was stirred at room temperature for 16 hours. LCMS showed the reaction was completed. The reaction mixture was concentrated under reduced pressure to obtain the crude product of compound **4-2** (7.70 g), which was directly used in the next step. LCMS m/z = 467.0 [M+1]⁺.

### Step 2: Preparation of compound 4-3

The crude product of compound **4-2** (7.70 g) was dissolved in acetonitrile (100 mL). The mixture was added with 1-chloromethyl-4-fluoro-1,4-diazoniabicyclo[2.2.2]octane bis(tetrafluoroborate) (7.50 g, 21.4 mmol) and sodium carbonate (2.27 g, 21.4 mmol). The reaction mixture was stirred at room temperature for 1 hour. LCMS showed the reaction was completed. The reaction mixture was poured into 1N hydrochloric acid solution (100 mL), stirred at room temperature for 10 minutes, and extracted with ethyl acetate (100 mL × 3). The combined organic phase was washed with saturated brine (50 mL × 3), dried over anhydrous sodium sulfate, filtered, and concentrated under reduced pressure, and the residue was purified by column chromatography to obtain compound **4-3** (1.50 g, two-step yield of 57.4%). ¹H NMR (400 MHz, DMSO-d₆) δ = 8.55 (d, *J* = 8.6 Hz, 1H), 8.24 (s, 1H), 7.90 (d, *J* = 7.4 Hz, 1H), 7.85 (s, 1H), 7.84 - 7.78 (m, 1H), 7.47 (d, *J* = 8.6 Hz, 1H), 5.54 (d, *J* = 49.9 Hz, 1H).

### Step 3: Preparation of compound 4-4

Compound **4-3** (1.00 g, 2.42 mmol) was dissolved in ethanol (20 mL). At -78°C, sodium borohydride (184 mg, 4.84 mmol) was added thereto. The reaction mixture was continued to react for 1 hour at this temperature. TLC showed the reaction was completed. The reaction mixture was poured into saturated ammonium chloride solution (50 mL), stirred for 10 minutes, and extracted with ethyl acetate (50 mL × 3). The combined organic phase was washed with saturated brine (50 mL × 3), dried over anhydrous sodium sulfate, filtered, and concentrated under reduced pressure, and the residue was purified by column chromatography to obtain compound **4-4** (670 mg, yield of 66.7%). ¹H NMR (400 MHz, DMSO-d₆) δ = 8.12 (d, *J* = 8.4 Hz, 1H), 7.73 (d, *J* = 8.3 Hz, 1H), 7.54 (s, 1H), 7.52 (s, 1H), 7.50 - 7.44 (m, 2H), 6.08 (d, *J* = 9.1 Hz, 1H), 5.51 - 5.27 (m, 2H).

### Step 4: Preparation of compound 4

To a solution of compound **4-4** (900 mg, 2.16 mmol) in 2-methyltetrahydrofuran (20 mL) was added dropwise a solution of diethylaminosulfur trifluoride (720 mg, 4.32 mmol) in 2-methyltetrahydrofuran (10 mL) at 0°C. The reaction mixture was continued to stir for 1 hour at this temperature. TLC showed the reaction was completed. The reaction mixture was poured into saturated sodium bicarbonate solution (20 mL), and extracted with ethyl acetate (50 mL × 3). The combined organic phase was washed with saturated brine (50 mL × 3), dried over anhydrous sodium sulfate, and concentrated under reduced pressure, and the residue was purified by column chromatography to obtain **compound 4** (350 mg, yield of 38.8%).

### Step 5: Preparation of compounds 4-P1, 4-P2, 4-P3, and 4-P4

Compound 4 (350 mg, 0.839 mmol) was subjected to preparative SFC (column DAICEL CHIRALPAK^{®}IB (250 * 25 mm, 10 µm)); mobile phase [A: carbon dioxide, B: MeOH (containing 0.1% diethylamine)]; B%: 30% to 30% to obtain compound 4-P1 (retention time of 2.217 minutes), a mixture of compound 4-P2 and compound 4-P3, and compound 4-P4 (retention time of 2.604 minutes). The retention time was determined by the following analytical method: column: DAICEL CHIRALPAK^{®}IB, 100 * 3.0 mm, 3 µm, mobile phase [A: carbon dioxide, B: methanol (containing 0.1% diethylamine)], 40% B, flow rate: 1.5 mL/min, column temperature: 35°C.

The mixture of compound 4-P2 and compound 4-P3 was subjected to preparative SFC (column DAICELCHIRALCEL^{®}OJ (250 * 25 mm, 10 µm)); mobile phase [A: carbon dioxide, B: methanol (containing 0.1% diethylamine)]; B%: 30% to 30%) to obtain compound 4-P2 (retention time of 2.443 minutes) and compound 4-P3 (retention time of 2.537 minutes). The retention time was determined by the following analytical method: column: DAICEL CHIRALPAK^{®}IB, 100 * 3.0 mm, 3 µm, mobile phase [A: carbon dioxide, B: methanol (containing 0.1% diethylamine)], 40% B, flow rate: 1.5 mL/ min, column temperature: 35°C.

Compound 4-P1 (7.32 mg, recovery rate of 2.09%), ¹H NMR (400 MHz, MeOD) δ = 8.02 (d, *J* = 8.5 Hz, 1H), 7.52 - 7.48 (m, 1H), 7.47 (d, *J* = 8.6 Hz, 1H), 7.44 (s, 1H), 7.37 (dt, *J* = 9.5, 2.3 Hz, 1H), 6.36 (ddd, *J* = 54.5, 10.2, 5.1 Hz, 1H), 5.47 (ddd, *J* = 50.7, 17.9, 5.1 Hz, 1H). Compound 4-P2 (4.88 mg, recovery rate of 1.39%), ¹H NMR (400 MHz, MeOD) δ = 8.02 (d, *J* = 8.5 Hz, 1H), 7.52 - 7.48 (m, 1H), 7.47 (d, *J* = 8.6 Hz, 1H), 7.44 (s, 1H), 7.37 (dt, *J* = 9.5, 2.3 Hz, 1H), 6.36 (ddd, *J* = 54.5, 10.2, 5.1 Hz, 1H), 5.47 (ddd, *J* = 50.7, 17.9, 5.1 Hz, 1H). Compound 4-P3 (117 mg, recovery rate of 33.4%), ¹H NMR (400 MHz, MeOD) δ = 8.06 (dd, *J* = 8.5, 2.0 Hz, 1H), 7.57 - 7.51 (m, 2H), 7.45 (dt, *J* = 9.3, 2.3 Hz, 1H), 7.37 (d, *J* = 8.5 Hz, 1H), 6.04 (dd, *J* = 49.4, 13.3 Hz, 1H), 5.63 (dd, *J* = 47.5, 5.8 Hz, 1H). Compound 4-P4 (119 mg, recovery rate of 34.0%), ¹H NMR (400 MHz, MeOD) δ = 8.06 (dd, *J* = 8.5, 2.0 Hz, 1H), 7.57 - 7.51 (m, 2H), 7.45 (dt, *J* = 9.3, 2.3 Hz, 1H), 7.37 (d, *J* = 8.5 Hz, 1H), 6.04 (dd, *J* = 49.4, 13.3 Hz, 1H), 5.63 (dd, *J* = 47.5, 5.8 Hz, 1H).

### Experimental example 1: Luciferase assay

786-O-HRE-Luc cells were obtained by infecting 786-O (ATCC^{®}CRL-1932TM) cells purchased from ATCC with commercial lentivirus. Appropriate 786-O-HRE-Luc single cell clones were screened, expanded, and used for the subsequent luciferase assay. For the luciferase assay, 100 × DMSO stock solution dissolved with the drug was prepared in assay medium (RPMI-1640 containing 2% FBS; FBS: 10099141C, Gibco; RPMI-1640: 12440053, Gibco) to formulate a series of dilutions of 10 × compounds. To a clear flat-bottom 96-well plate (3599, Corning) was added 20 µL of the 10 × compound dilution. Then approximately 100,000 786-O-HRE-Luc cells in 180 µL of culture medium were seeded into the 96-well plate. The final concentration of DMSO (D2650, Sigma) in each well was 0.1%. After approximately 24 hours of incubation in the incubator, luciferase activity was determined using the Dual-Luciferase^{®} Reporter Assay System (E1960, Promega) reagent following the manufacturer's recommended method. EC₅₀ values were calculated by GraphPad Prism software using the dose-response-inhibition (four parameter) equation. The experimental results are shown in Table 1.

**Table 1. EC₅₀ values of selected compounds in luciferase assay**

| Compound number | Luciferase EC₅₀ (nM) |
|---|---|
| Compound 2 | 46 |
| Compound 3-P1 | 67.7 |
| Compound 3-P2 | 3991 |
| Compound 3-P3 | 12.2 |
| Compound 3-P4 | 6411 |
| Compound 4-P1 | 19.2 |
| Compound 4-P2 | 48.5 |
| Compound 4-P3 | 6.1 |
| Compound 4-P4 | 511 |
| Example 19 of US9796697B2 | 59.7 |

As can be seen from the experimental results in Table 1, the compounds of the present disclosure have excellent in vitro activity and can inhibit the luciferase level of HIF response element(HRE)-dependent expression.

### Experimental example 2: VEGF ELISA assay

100 × DMSO stock solution dissolved with the drug was prepared in assay medium (RPMI-1640 containing 2% FBS; FBS: 10099141C, Gibco; RPMI-1640: 12440053, Gibco) to formulate a series of dilutions of 10 × compounds. To a clear flat-bottom 96-well plate (3599, Corning) was added 20 µL of the 10 × compound dilution. Then approximately 40,000 786-O cells (ATCC^{®}CRL-1932TM) in 180 µL of culture medium were seeded into the 96-well plate. The final concentration of DMSO (D2650, Sigma) in each well was 0.1%. After incubating in the incubator for about 48 hours, 100 µL of the upper medium from each well was pipetted and placed in a new 96-well plate (3799, Corning). An ELISA kit (DY293B, R&D Systems) was used. The concentration of VEGF was determined according to the OD value of each well at 450 nM detected by a microplate reader. EC₅₀ values were calculated by GraphPad Prism software using the dose-response-inhibition (four parameter) equation. The experimental results are shown in Table 2.

**Table 2. EC₅₀ values of selected compounds in VEGF ELISA assay**

| Compound number | VEGF ELISA EC₅₀ (nM) |
|---|---|
| Compound 1 | 115 |
| Compound 3-P1 | 180.4 |
| Compound 3-P2 | 2765 |
| Compound 3-P3 | 30.9 |
| Compound 3-P4 | 4922 |
| Compound 4-P1 | 40.5 |
| Compound 4-P2 | 54.4 |
| Compound 4-P3 | 15.2 |
| Compound 4-P4 | 736.6 |
| Example 19 of US9796697B2 | 165.9 |

As can be seen from the experimental results in Table 2, the compounds of the present disclosure have the activity of obviously inhibiting the expression of VEGF.

### Experimental Example 3: Pharmacokinetic test

### 1. Experimental purpose

SD rats were taken as the experimental animals, and compound 3-P3 and comparative example PT2385 were given by gavage, and the drug concentration in plasma at various time points was measured by LC-MS/MS method, and the pharmacokinetic characteristics of the compound of the present disclosure and the compound of the comparative example in rats were studied.

### 2. Experimental scheme

### 2.1 Experimental drugs and animals

Experimental drugs: compound 3-P3 and comparative example PT2385;
animals: SD rats, male, 200 to 220 g, purchased from Shanghai Jiesijie Laboratory Animal Co., Ltd.

### 2.2 Drug formulation

An appropriate amount of compound 3-P3 was weighed, added with an appropriate amount of 10% ethanol + 30% polyethylene glycol 400 + 60% (0.5% sodium carboxymethylcellulose + 0.5% Tween 80) to prepare a 1 mg/mL suspension by vortex oscillation and ultrasound. An appropriate amount of compound 3-P3 was weighed, added with an appropriate amount of 10% ethanol + 30% polyethylene glycol 400 + 60% (0.5% sodium carboxymethylcellulose + 0.5% Tween 80) to prepare a 0.5 mg/mL suspension by vortex oscillation and ultrasound.

### 2.3 Administration

The SD rats in each test compound group (3 rats in each group) were fasted overnight and then intragastrically administered the compounds (PO, administration dosage of 10 mg/kg or 5 mg/kg, administration volume of 10 mL/kg), and given food 4 hours after administration.

### 3. Procedures

Approximately 0.2 mL of blood was collected from the jugular vein before administration and 0.083 hours, 0.25 hours, 0.5 hours, 1 hour, 2 hours, 4 hours, 6 hours, 8 hours, and 24 hours after administration, anticoagulated with sodium heparin. Blood samples were placed on ice after collection, and plasma was separated by centrifugation (centrifugation conditions: 1500 g, 10 minutes). The collected plasma was stored at -40°C to -20°C before analysis.

LC-MS/MS was used to determine the content of the test compound in rat plasma after intragastric administration.

### 4. Pharmacokinetic parameter results

The pharmacokinetic parameters of the compound 3-P3 of the present disclosure and the comparative example PT2385 are shown in Table 3.

**Table 3 Pharmacokinetic results**

| Compound number | Pharmacokinetic experiment | | |
|---|---|---|---|
| | Administration mode | Plasma concentration | Area under the curve |
| | Administration dosage | | |
| | | Cmax | AUC_{0-∞} |
| | | (ng/mL) | (ng * h/mL) |
| Compound 3-P3 | PO (10 mg/kg) | 4646 | 24806 |
| Comparative example PT2385 | PO (5 mg/kg) | 193 | 843 |

Conclusion: Compared with the comparative example PT2385, compound 3-P3 has significantly improved plasma concentration and area under the curve.

All references mentioned in the present disclosure are incorporated by reference in the present disclosure as if each were individually incorporated by reference. In addition, it should be understood that after reading the above teaching content of the present disclosure, those skilled in the art can make various changes or modifications to the present disclosure, and these equivalent forms also fall within the scope defined by the claims of the present disclosure.

## Claims

1. A compound of formula (I), an optical isomer thereof, or a pharmaceutically acceptable salt thereof, 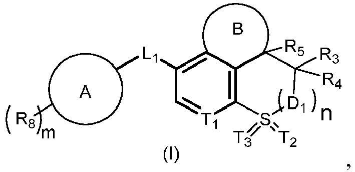
wherein ring A is selected from C₄₋₆ cycloalkyl, 4- to 6-membered heterocycloalkyl, phenyl, and 5- to 6-membered heteroaryl;
ring B is selected from C₅₋₆ cycloalkyl, 5- to 6-membered heterocyclyl, and 5- to 6-membered cycloalkenyl, and the C₅₋₆ cycloalkyl, 5- to 6-membered heterocyclyl, or 5- to 6-membered cycloalkenyl is optionally substituted by 1, 2, 3, or 4 R;
L₁ is selected from a single bond, -O-, -S-, and -N(R_{L})-;
T₁ is selected from -C(R_{T})- and -N-;
T₂ is selected from O, =NR₉, or T₂ is absent;
T₃ is selected from =NR₁₀ and O;
D₁ is independently selected from -C(R_{D1})₂- and -N(R_{D1})-;
R₃, R₄ are each independently selected from H, F, Cl, Br, and I;
R₅ is selected from H, OH, F, and NH₂;
Rs is independently selected from H, F, Cl, Br, I, CN, C₁₋₆ alkyl, and C₁₋₆ alkoxy, and the C₁₋₆ alkyl or C₁₋₆ alkoxy is optionally substituted by 1, 2, or 3 R₈ₐ;
R₉ is selected from H, CN, OH, C₁₋₆ alkyl, and C₃₋₆ cycloalkyl, and the C₁₋₆ alkyl and C₃₋₆ cycloalkyl are optionally substituted by 1, 2, or 3 R;
R₁₀ is selected from H, CN, OH, C₁₋₆ alkyl, and C₃₋₆ cycloalkyl, and the C₁₋₆ alkyl and C₃₋₆ cycloalkyl are optionally substituted by 1, 2, or 3 R;
R, R_{T}, R_{D1}, R_{L}, R₈ₐ are each independently selected from H, halogen, OH, NH₂, CN, 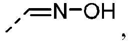 C₁₋₆ alkyl, C₁₋₆ alkoxy, and C₂₋₆ alkenyl, and the C₁₋₆ alkyl, C₁₋₆ alkoxy, or C₂₋₆ alkenyl is optionally substituted by 1, 2, or 3 R';
R' is independently selected from H, halogen, OH, NH₂, CN, and C₁₋₆ alkyl;
m is independently 0, 1, 2, 3, or 4;
n is independently 0, 1, 2, or 3;
the 4- to 6-membered heterocycloalkyl, 5- to 6-membered heterocyclyl, or 5- to 6-membered heteroaryl contains 1, 2, or 3 heteroatoms or heteroatom groups independently selected from -O-, -NH-, -S-, -C(=O)-, -C(=O)O-, -S(=O)-, -S(=O)₂-, and N.

2. A compound of formula (II), an optical isomer thereof, or a pharmaceutically acceptable salt thereof, 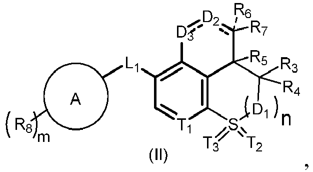
wherein ring A is selected from C₄₋₆ cycloalkyl, 4- to 6-membered heterocycloalkyl, phenyl, and 5- to 6-membered heteroaryl;
L₁ is selected from a single bond, -O-, -S-, and -N(R_{L})-;
T₁ is selected from -C(R_{T})- and -N-;
T₂ is selected from O, =NR₉, or T₂ is absent;
T₃ is selected from =NR₁₀ and O;
D₁ is independently selected from -C(R_{D1})₂- and -N(R_{D1})-;
when 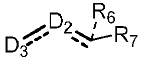 is 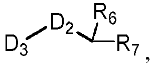 D₂ and D₃ are each independently selected from a single bond, -O-, -N(R)-, -C(R)₂-, -C(=R)-, -C(=O)-, and -C(=NR)-, and R₆, R₇ are each independently selected from H, F, Cl, Br, and I;
when 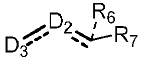 is 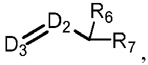 D₂ and D₃ are each independently selected from - C(R)- and N, and R₆, R₇ are each independently selected from H, F, Cl, Br, and I;
when 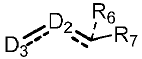 is 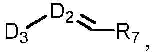 D₂ is independently selected from -C(R)- and N, D₃ is independently selected from a single bond, -O-, -N(R)-, -C(R)₂-, -C(=R)-, -C(=O)-, and - C(=NR)-, and R₇ is independently selected from H, F, Cl, Br, and I;
R₃, R₄ are each independently selected from H, F, Cl, Br, and I;
R₅ is selected from H, OH, F, and NH₂;
R₈ is independently selected from H, F, Cl, Br, I, CN, C₁₋₆ alkyl, and C₁₋₆ alkoxy, and the C₁₋₆ alkyl or C₁₋₆ alkoxy is optionally substituted by 1, 2, or 3 R₈ₐ;
R₉ is selected from H, CN, OH, C₁₋₆ alkyl, and C₃₋₆ cycloalkyl, and the C₁₋₆ alkyl and C₃₋₆ cycloalkyl are optionally substituted by 1, 2, or 3 R;
R₁₀ is selected from H, CN, OH, C₁₋₆ alkyl, and C₃₋₆ cycloalkyl, and the C₁₋₆ alkyl and C₃₋₆ cycloalkyl are optionally substituted by 1, 2, or 3 R;
R, R_{T}, R_{D1}, R_{L}, R₈ₐ are each independently selected from H, halogen, OH, NH₂, CN, 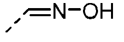 C₁₋₆ alkyl, C₁₋₆ alkoxy, and C₂₋₆ alkenyl, and the C₁₋₆ alkyl, C₁₋₆ alkoxy, or C₂₋₆ alkenyl is optionally substituted by 1, 2, or 3 R';
R' is independently selected from H, halogen, OH, NH₂, CN, and C₁₋₆ alkyl;
m is independently 0, 1, 2, 3, or 4;
n is independently 0, 1, 2, or 3;
the 4- to 6-membered heterocycloalkyl or 5- to 6-membered heteroaryl contains 1, 2, or 3 heteroatoms or heteroatom groups independently selected from -O-, -NH-, -S-, -C(=O)-, - C(=O)O-, -S(=O)-, -S(=O)₂-, and N.

3. The compound, the optical isomer thereof, or the pharmaceutically acceptable salt thereof according to claim 1, wherein the compound has a structure of formula (II-A) or formula (II-B), 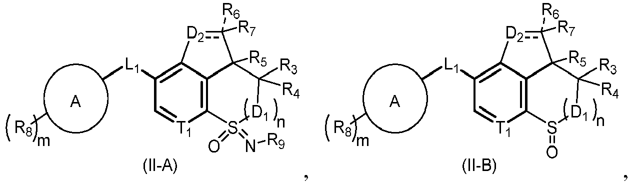
wherein ring A is selected from C₄₋₆ cycloalkyl, 4- to 6-membered heterocycloalkyl, phenyl, and 5- to 6-membered heteroaryl;
L₁ is selected from a single bond, -O-, -S-, and -N(R_{L})-;
T₁ is selected from -C(R_{T})- and -N-;
D₁ is independently selected from -C(R_{D1})₂- and -N(R_{D1})-;
when 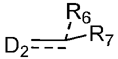 is 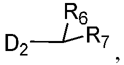 D₂ is selected from -O-, -N(R)-, -C(R)₂-, -C(=R)-, - C(=O)-, and -C(=NR)-, and R₆, R₇ are each independently selected from H, F, Cl, Br, and I;
when 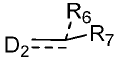 is 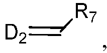 D₂ is selected from -C(R)- and N, and R₇ is selected from H, F, Cl, Br, and I;
R₃, R₄ are each independently selected from H, F, Cl, Br, and I;
R₅ is selected from H, OH, F, and NH₂;
R₈ is independently selected from H, F, Cl, Br, I, CN, C₁₋₆ alkyl, and C₁₋₆ alkoxy, and the C₁₋₆ alkyl or C₁₋₆ alkoxy is optionally substituted by 1, 2, or 3 R₈ₐ;
R₉ is selected from H, CN, OH, C₁₋₆ alkyl, and C₃₋₆ cycloalkyl, and the C₁₋₆ alkyl and C₃₋₆ cycloalkyl are optionally substituted by 1, 2, or 3 R;
R, R_{T}, R_{D1}, R_{L}, R₈ₐ are each independently selected from H, halogen, OH, NH₂, CN, 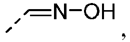 C₁₋₆ alkyl, C₁₋₆ alkoxy, and C₂₋₆ alkenyl, and the C₁₋₆ alkyl, C₁₋₆ alkoxy, or C₂₋₆ alkenyl is optionally substituted by 1, 2, or 3 R';
R' is independently selected from H, halogen, OH, NH₂, CN, and C₁₋₆ alkyl;
m is independently 0, 1, 2, 3, or 4;
n is independently 0, 1, 2, or 3;
the 4- to 6-membered heterocycloalkyl or 5- to 6-membered heteroaryl contains 1, 2, or 3 heteroatoms or heteroatom groups independently selected from -O-, -NH-, -S-, -C(=O)-, - C(=O)O-, -S(=O)-, -S(=O)₂-, and N.

4. The compound, the optical isomer thereof, or the pharmaceutically acceptable salt thereof according to claim 1, wherein the compound has a structure of formula (III-A), 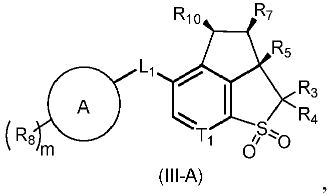
wherein ring A is selected from C₄₋₆ cycloalkyl, 4- to 6-membered heterocycloalkyl, phenyl, and 5- to 6-membered heteroaryl;
L₁ is selected from a single bond, -O-, -S-, and -N(R_{L})-;
T₁ is selected from -C(R_{T})- and -N-;
R₇, R₁₀ are each independently selected from H, F, Cl, Br, and I;
R₃, R₄ are each independently selected from H, F, Cl, Br, and I;
R₅ is selected from H, OH, F, and NH₂;
R₅ is independently selected from H, F, Cl, Br, I, CN, C₁₋₆ alkyl, and C₁₋₆ alkoxy, and the C₁₋₆ alkyl or C₁₋₆ alkoxy is optionally substituted by 1, 2, or 3 R₈ₐ;
R_{T}, R_{L}, R₈ₐ are each independently selected from H, halogen, OH, NH₂, CN, 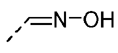 , C₁₋₆ alkyl, C₁₋₆ alkoxy, and C₂₋₆ alkenyl, and the C₁₋₆ alkyl, C₁₋₆ alkoxy, or C₂₋₆ alkenyl is optionally substituted by 1, 2, or 3 R';
R' is independently selected from H, halogen, OH, NH₂, CN, and C₁₋₆ alkyl;
m is independently 0, 1, 2, 3, or 4;
the 4- to 6-membered heterocycloalkyl or 5- to 6-membered heteroaryl contains 1, 2, or 3 heteroatoms or heteroatom groups independently selected from -O-, -NH-, -S-, -C(=O)-, - C(=O)O-, -S(=O)-, -S(=O)₂-, and N.

5. The compound, the optical isomer thereof, or the pharmaceutically acceptable salt thereof according to any one of claims 1 to 4, wherein R₈ is selected from H, F, Cl, Br, I, and CN;
optionally, ring A is selected from phenyl, pyridyl, pyridazinyl, cyclobutyl, cyclopentyl, and cyclohexyl.

6. The compound, the optical isomer thereof, or the pharmaceutically acceptable salt thereof according to any one of claims 1 to 4, wherein the structural moiety 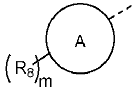 is selected from

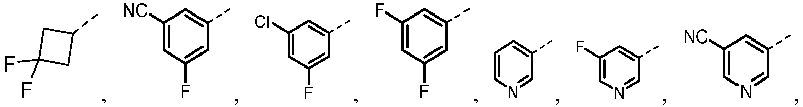

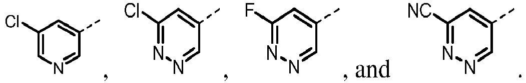

7. The compound, the optical isomer thereof, or the pharmaceutically acceptable salt thereof according to any one of claims 1, 2, or 3, wherein R, R_{T}, R_{D1}, R_{L}, R₈ₐ are independently selected from H, CH₃, F, Cl, Br, I, CN, OH,

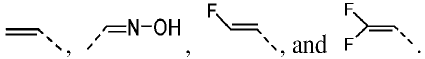

8. The compound, the optical isomer thereof, or the pharmaceutically acceptable salt thereof according to claim 1, wherein ring B is selected from cyclopentyl, cyclopentenyl, cyclohexyl, cyclohexenyl, oxocyclohexyl, tetrahydro-2H-pyran-2-keto, piperidin-2-keto, tetrahydro-2H-pyranyl, and piperidinyl, and the cyclopentyl, cyclopentenyl, cyclohexyl, cyclohexenyl, oxocyclohexyl, tetrahydro-2H-pyran-2-keto, piperidin-2-keto, tetrahydro-2H-pyranyl, or piperidinyl is optionally substituted by 1, 2, 3, or 4 R.

9. The compound, the optical isomer thereof, or the pharmaceutically acceptable salt thereof according to claim 1, wherein ring B is selected from 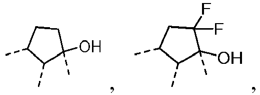 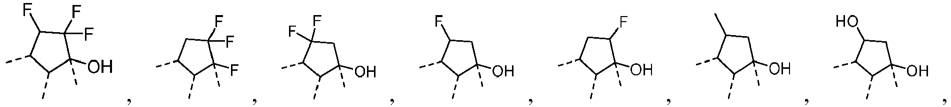 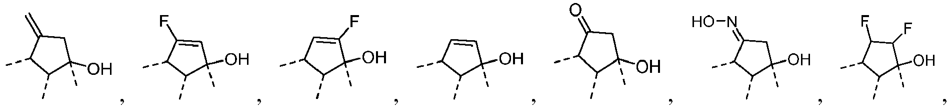 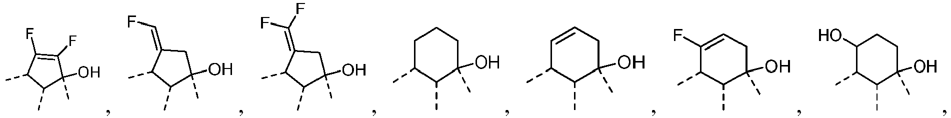 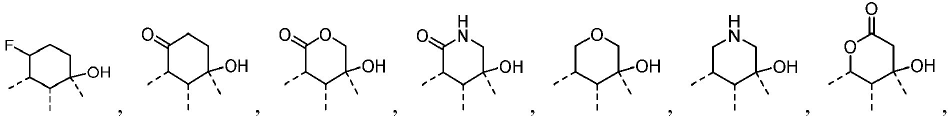 and 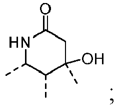 optionally, ring B is selected from

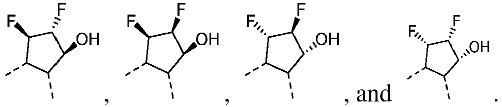

10. The compound, the optical isomer thereof, or the pharmaceutically acceptable salt thereof according to claim 1 or 2, wherein the structural moiety 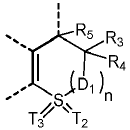 is selected from

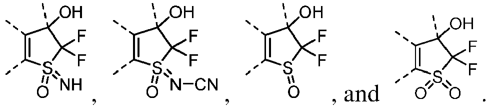

11. The compound, the optical isomer thereof, or the pharmaceutically acceptable salt thereof according to claim 1, wherein the structural moiety 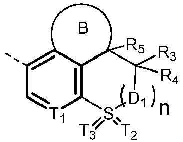 is selected from

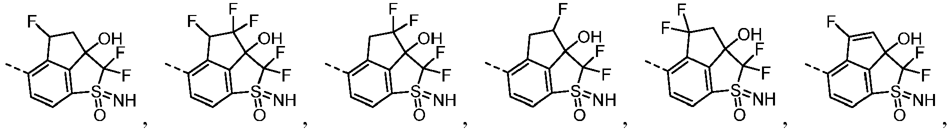

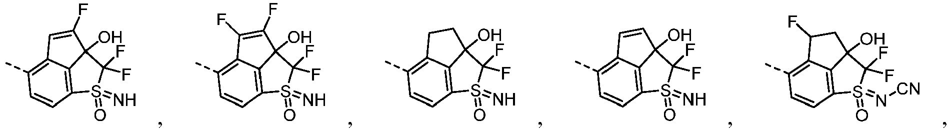

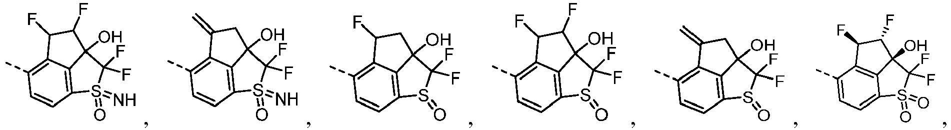

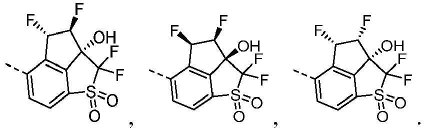

12. A compound of the following formula, an optical isomer thereof, or a pharmaceutically acceptable salt thereof, selected from:

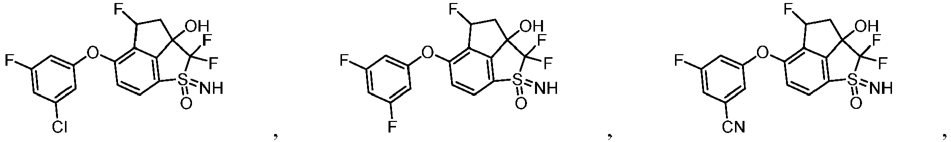

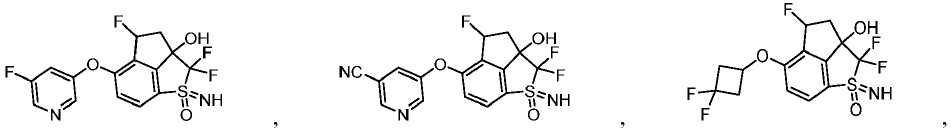

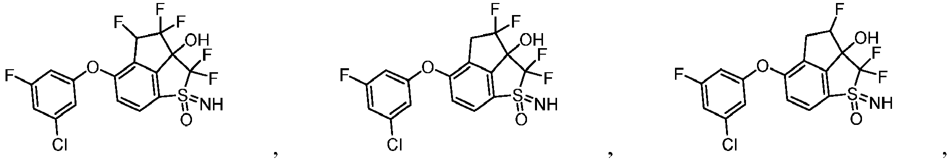

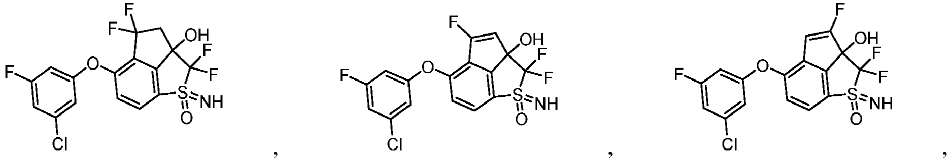

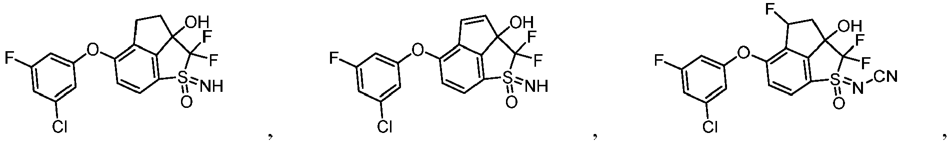

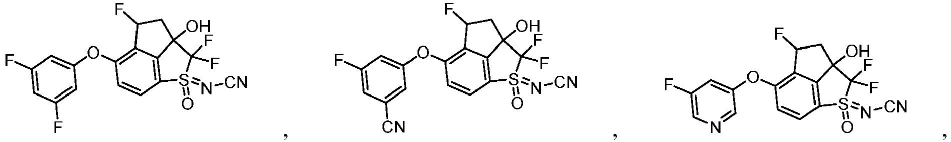

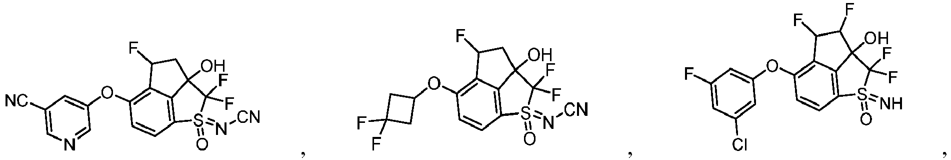

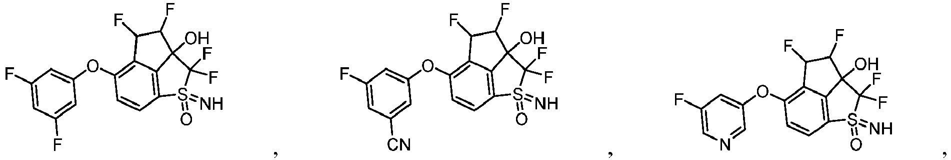

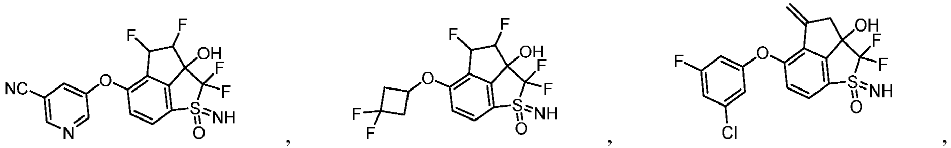

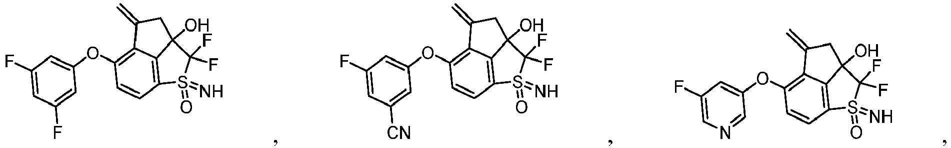

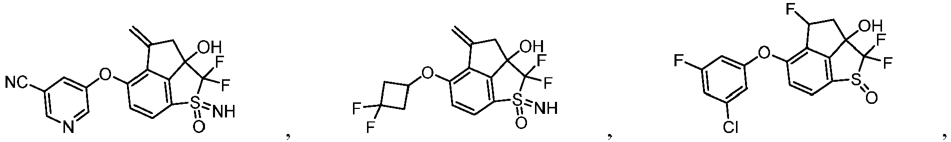

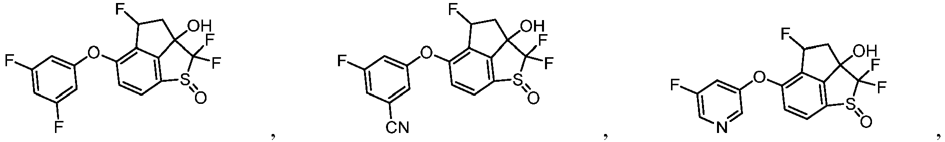

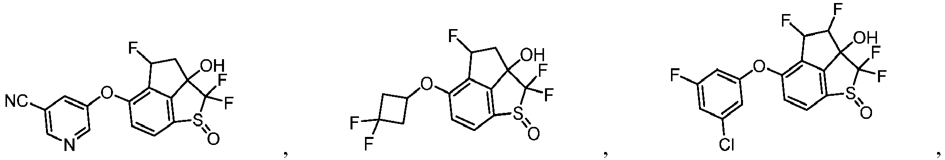

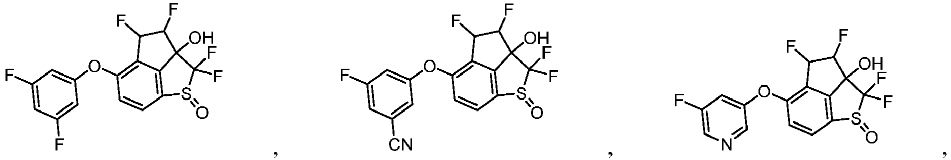

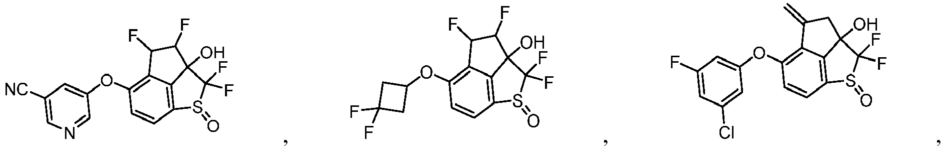

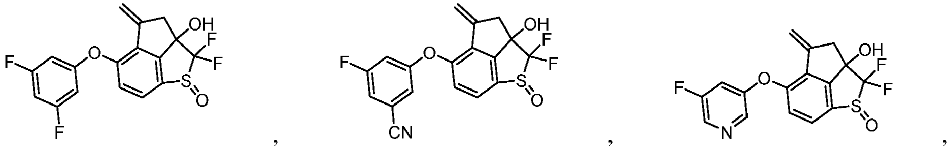

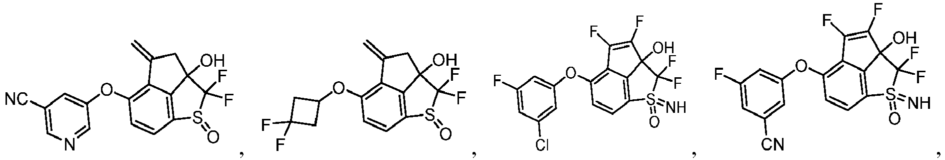

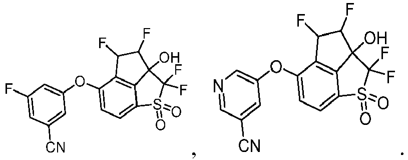

13. A compound of the following formula, an optical isomer thereof, or a pharmaceutically acceptable salt thereof, selected from:

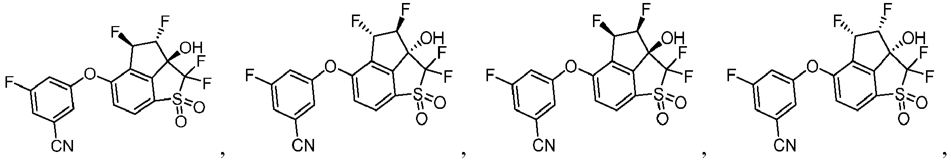

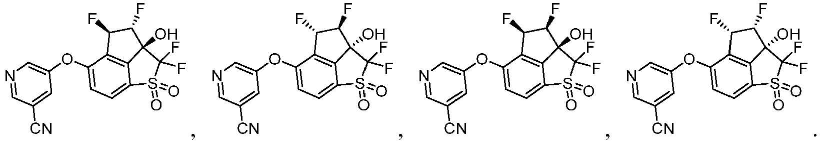

14. A use of the compound or the pharmaceutically acceptable salt thereof according to any one of claims 1 to 13 in the manufacture of a medicament for the prevention or treatment of HIF2α-mediated diseases.

15. The use according to claim 14, wherein the HIF2α-mediated diseases comprise renal cancer, brain glioma, Von Hippel-Lindau syndrome, lung cancer, colorectal cancer, ovarian cancer, breast cancer, cervical cancer, gastric cancer, liver cancer, thyroid cancer, multiple myeloma, pancreatic ductal carcinoma, lung squamous cell carcinoma, colon cancer, hemangioma, pulmonary hypertension, and inflammatory bowel disease.
